# EUROPEAN PATENT SPECIFICATION

(11) **EP 4 058 583 B1**
(45) Date of publication and mention of the grant of the patent: **04.11.2026**
(21) Application number: 20803847.1
(22) Date of filing: 12.11.2020
(51) Int. Cl.: C12N 15/63, C12N 9/02, C12N 9/78

(54) **METHODS OF CELL SELECTION**
VERFAHREN ZUR ZELLSELEKTION
PROCÉDÉ DE SÉLECTION DE CELLULE

(30) Priority: 14.11.2019 EP 19209232; 14.07.2020 EP 20185798
(43) Date of publication of application: 21.09.2022
(73) Proprietor: Lonza Ltd, 3930 Visp (CH)
(72) Inventor: JAQUES, Colin, 4052 Basel (CH); BUDGE, James, 4052 Basel (CH); ROBOOL, Joanne, 4052 Basel (CH); SMALES, Christopher, 4052 Basel (CH)
(74) Representative: Greiner, Elisabeth
(86) International application number: PCT/EP2020/081923
(87) International publication number: WO 2021/094461

(56) References cited:
- WO-A1-2017/167866
- WO-A1-2018/167621
- DAUBNER S.COLETTE ET AL: "Expression and Characterization of the Catalytic Domain of Human Phenylalanine Hydroxylase", ARCHIVES OF BIOCHEMISTRY AND BIOPHYSICS, vol. 348, no. 2, 1 December 1997 (1997-12-01), US, pages 295 - 302, XP093111133, ISSN: 0003-9861, Retrieved from the Internet <URL:https://pdf.sciencedirectassets.com/272380/1-s2.0-S0003986100X00999/1-s2.0-S0003986197904355/main.pdf?X-Amz-Security-Token=IQoJb3JpZ2luX2VjEKD//////////wEaCXVzLWVhc3QtMSJHMEUCIGX7U9lACO+AFLdShx3KTC5ZhXS/gmmDjJEl9lqAn1fuAiEA3s4T8ZO7QvUh+gySe+CMec68XrWpZn/l90X7/fX15AAqsgUIGRAFGgwwNTkwMDM1NDY4NjUiDItwd> DOI: 10.1006/abbi.1997.0435
- KNAPPSKOG PER M. ET AL: "Structure/Function Relationships in Human Phenylalanine Hydroxylase : Effect of Terminal Deletions on the Oligomerization, Activation and Cooperativity of Substrate Binding to the Enzyme", EUROPEAN JOURNAL OF BIOCHEMISTRY, vol. 242, no. 3, 1 December 1996 (1996-12-01), pages 813 - 821, XP093111136, ISSN: 0014-2956, DOI: 10.1111/j.1432-1033.1996.0813r.x
- R CHRISTENSEN ET AL: "Development of a skin-based metabolic sink for phenylalanine by overexpression of phenylalanine hydroxylase and GTP cyclohydrolase in primary human keratinocytes", GENE THERAPY, vol. 7, no. 23, 1 December 2000 (2000-12-01), GB, pages 1971 - 1978, XP055687529, ISSN: 0969-7128, DOI: 10.1038/sj.gt.3301337
- LAUFS S ET AL: "RECONSTITUTION OF A METABOLIC PATHWAY WITH TRIPLE-CISTRONIC IRES-CONTAINING RETROVIRAL VECTORS FOR CORRECTION OF TETRAHYDROBIOPTERIN DEFICIENCY", JOURNAL OF GENE MEDICINE, JOHN WILEY & SONS, INC, US, vol. 2, no. 1, 1 January 2000 (2000-01-01), pages 22 - 31, XP009040200, ISSN: 1099-498X, DOI: 10.1002/(SICI)1521-2254(200001/02)2:1<22::AID-JGM86>3.0.CO;2-1
- ZHAOBING DING ET AL: "Correction of Murine PKU Following AAV-mediated Intramuscular Expression of a Complete Phenylalanine Hydroxylating System", MOLECULAR THERAPY, vol. 16, no. 4, 1 April 2008 (2008-04-01), US, pages 673 - 681, XP055755277, ISSN: 1525-0016, DOI: 10.1038/mt.2008.17

## Description

### FIELD OF THE INVENTION

The present disclosure relates to methods and compositions for identifying, selecting, or culturing cells comprising a subject nucleic acid sequence.

### BACKGROUND

Cell expression systems are commonly used for the production of recombinant biological products, such as therapeutic biologics. The development of production line cells involves introducing nucleic acid constructs encoding recombinant products of interest into host cells and selecting for cells that contain these nucleic acid constructs. This generally involves subjecting the cells to a selection pressure to favour cells that have taken up the foreign nucleic acids. Early selectable marker systems used antibiotic resistance markers but there has been a trend away from the use of such systems. Some alternative systems are based on complementing metabolic deficiencies e.g. dihydrofolate reductase (DHFR) and glutamine synthetase (GS).

WO 2017/167866 relates to recombinant microbial host cells comprising biosynthetic pathways and their use in producing oxidation products of aromatic amino acids, such as 5HTP, as well as downstream products such as melatonin and related compounds.

Christensen et al. (Gene Therapy, vol. 7, no. 23, December 1, 2000) describes the development of a skin-based metabolic sink for phenylalanine by overexpression of phenylalanine hydroxylase and GTF cyclohydrolase in primary human keratinocytes.

Laufs et al. (Journal of Gene Medicine, vol. 2, no. 1, January 1, 2000) describes reconstitution of a metabolic pathway with triple-cistronic IRES-containing retroviral vectors for correction of tetrahydrobiopterin deficiency.

WO 2018/167621 relates to cells having tyrosine prototrophy and to methods of selecting cells that contain one or more exogenous nucleic acid constructs by selecting cells which exhibit tyrosine prototrophy. The exogenous nucleic acid may contain one or both of the PAH and PCBD1 genes, and a nucleotide sequence of interest for expression in the host cell.

Zhaobing et al. (Molecular Therapy, 16, no. 4, April 1, 2008) describes correction of murine PKU following AAV-mediated expression of a complete phenylalanine hydroxylating system.

Daubner et al. (Archives of Biochemistry and Biophysics, vol. 348, no. 2, December 1, 1997) relates to the catalytic domain of human phenylalanine hydroxylase and describes the expression and characterization of a truncated version of human phenylalanine hydroxylase which contains the carboxy terminal 336 amino acids.

Knappskog et al. (European Journal of Biochemistry, vol. 242, no. 3, December 1, 1996) describes structure/function relationships in human phenylalanine hydroxylase, including the effect of terminal deletions on the oligomerization, activation and cooperativity of substrate binding to the enzyme.

There remains however a need for new selection systems that can be used to select cells used to produce recombinant biological products. Further, since production host cell engineering strategies are increasingly being used, those strategies that involve the introduction of new sequences that modify host cell characteristics would benefit from a selection system that is separate from existing or future selection systems used for the introduction of sequences encoding biological products.

### SUMMARY OF THE INVENTION

The present invention relates to a tyrosine auxotrophy-based selection system and the manufacture of recombinant products without the need to include tyrosine in the media. We have found that a system based on phenylalanine hydroxylase (PAH - which catalyzes the conversion of phenylalanine to tyrosine) alone is not effective and that it is necessary to include a second enzyme related to tyrosine biosynthesis, namely GTP cyclohydrolase 1 (GCH1). Further we have found that use of PAH with a truncation that removes the N-terminal regulatory domain provides a significant advantage compared with the full length enzyme. Use of full length CHO PAH resulted in either no recovery in tyrosine-free medium after transfection or a much slower recovery time, whereas a truncated (tPAH) version of the molecule allowed for good recovery. The combination of PAH and GCH1 allows a cell to grow at a lower level of (e.g., in the absence of) tyrosine than a similar cell not expressing these enzymes.

Accordingly, in a first aspect, the present invention provides a method of selecting a eukaryotic cell comprising a nucleic acid sequence encoding a product of interest, the method comprising:
i) contacting a population of cells that are unable to survive or grow in the absence of tyrosine, with a vector system comprising
   a) a first nucleic acid sequence comprising a sequence encoding a phenylalanine hydroxylase (PAH) which lacks a functional N-terminal regulatory domain such that the PAH enzyme molecule is constitutively active, operably linked to a first control sequence which enables expression of the PAH in a host cell, wherein the PAH is CHO PAH or human PAH;
   b) a second nucleic acid sequence comprising a sequence encoding a GTP cyclohydrolase 1 (GCH1) operably linked to a second control sequence which enables expression of the GCH1 in a host cell; and
   c) a third nucleic acid sequence comprising a sequence encoding a product of interest operably linked to a third control sequence which enables expression of the product in a host cell,
   wherein
   (1.) (a), (b) and (c) are present in the same vector; or
   (2.) the vector system comprises two vectors wherein (c) is present in the same vector as (a) or (b),
   under conditions that permit uptake of the vector system by the cells;
ii) culturing the cells in the presence of phenylalanine and under conditions where the level of tyrosine is lower than the level required for survival or growth of cells that do not express the PAH and GCH1 enzymes encoded by the vector system; and
iii) selecting one or more cells that are able to grow under such conditions to obtain one or more cells which contain the nucleic acid sequence encoding the product.

Such vectors may be introduced into host cells, and those cells containing the vectors selected under tyrosine-limiting conditions which do not allow for efficient growth of non-transformed cells. Accordingly in a second aspect, the present invention provides a eukaryotic host cell comprising:
a) a first exogenous nucleic acid comprising a sequence which encodes a phenylalanine hydroxylase (PAH) which lacks a functional N-terminal regulatory domain such that the PAH enzyme molecule is constitutively active, operably linked to a first control sequence which enables expression of the PAH in the host cell, wherein the PAH is CHO PAH or human PAH;
b) a second exogenous nucleic acid which encodes a GTP cyclohydrolase 1 (GCH1), operably linked to a second control sequence which enables expression of the GCH1 in the host cell; and
c) a third exogenous nucleic acid which encodes a recombinant product of interest, operably linked to a third control sequence which enables expression of the recombinant product in the host cell, which third exogenous nucleic acid is present in the same exogenous nucleic acid sequence as the first and/or second exogenous nucleic acid.

The vector system of the present invention is typically used to select cells that have been successfully transformed with a nucleic acid encoding a product of interest, such as a recombinant polypeptide.

The level of tyrosine is selected to ensure a stringent selection and is supplemented with phenylalanine. In one embodiment the culture media includes no added tyrosine.

In a related aspect the present invention provides a vector system comprising one or more nucleic acid vectors comprising:
a) a first nucleic acid sequence comprising a sequence encoding a phenylalanine hydroxylase (PAH) which lacks a functional N-terminal regulatory domain such that the PAH enzyme molecule is constitutively active, operably linked to a first control sequence which enables expression of the PAH in a host cell, wherein the PAH is CHO PAH or human PAH;
b) a second nucleic acid sequence comprising a sequence encoding a GTP cyclohydrolase 1 (GCH1) operably linked to a second control sequence which enables expression of the GCH1 in a host cell; and
c) a multiple cloning site for inserting a sequence encoding a product of interest operably linked to a third control sequence which enables expression of the product in a host cell, wherein the multiple cloning site and third control sequence are present in the same vector as (a) and/or (b).

In another aspect the present invention provides a vector system comprising one or more nucleic acid vectors comprising:
a) a first nucleic acid sequence comprising a sequence encoding a phenylalanine hydroxylase (PAH) which lacks a functional N-terminal regulatory domain such that the PAH enzyme molecule is constitutively active, operably linked to a first control sequence which enables expression of the PAH in a host cell, wherein the PAH is CHO PAH or human PAH;
b) a second nucleic acid sequence comprising a sequence encoding a GTP cyclohydrolase 1 (GCH1) operably linked to a second control sequence which enables expression of the GCH1 in a host cell; and
c) a third nucleic acid sequence comprising a sequence encoding an antibody operably linked to a third control sequence which enables expression of the product in a host cell, which third nucleic acid sequence is present in the same vector as (a) and/or (b).

The selected host cells of the present invention comprising a nucleic acid sequence encoding the product of interest will typically be used in the manufacturing of that product. Accordingly in another aspect, the present invention provides a method of making a product, the method comprising culturing a host cell as claimed under conditions suitable for expressing the product, and recovering the product, and optionally subjecting the recovered product to one or more treatment or purification steps, wherein the cells are cultured in the presence of phenylalanine and under conditions where the level of tyrosine is lower than the level required for survival or growth of cells that do not express the PAH and GCH1 enzymes encoded by the vector system as claimed.

When a cell line developed using the host cells and selection processes of the present invention is used in large scale manufacturing, it may no longer be essential to exert a selection pressure by omitting tyrosine during culturing steps. However, tyrosine, which is considered an essential amino acid, has after cysteine the second lowest solubility in water of any of the amino acids. The low solubility of tyrosine can be a challenge for generating feed solutions of sufficient concentration to support culture of cells under biomanufacturing conditions, e.g., in fed-batch bioprocesses, e.g., in a bioreactor.

The host cells of the present invention can be efficiently grown in lower levels of (including in the absence of) tyrosine and reducing the need for high concentration tyrosine feed solutions. Since phenylalanine is consumed by the cells to produce tyrosine, the culture medium is supplemented with phenylalanine.

### BRIEF DESCRIPTION OF THE FIGURES

**Fig. 1** shows a schematic of the PAH enzyme's domain structure.
**Fig. 2** shows (A) histograms obtained using flow cytometry of the mean fluorescence from the population of cells after transfection and recovery for 3 weeks of the same CHO cell pools and (B) tables of the fluorescence data.
**Fig. 3** shows a graph of PAH mRNA amounts relative to control as measured by qRT-PCR.
**Fig. 4** shows (A) a graph of cell growth by viable cell concentration of various cell pools, some over-expressing truncated PAH, in the absence of tyrosine or glutamine, optionally supplemented with phenylalanine, over 18 days; and (B) a graph of culture viability of the same cell pools under the same conditions.
   **Fig. 5** shows growth characteristics of tyrosine prototrophic cell pools with varied phenylalanine supplementation.
**Fig. 6****:** shows graphs of growth characteristics of tyrosine prototrophic cell pools in CD CHO no tyrosine with 6 mM phenylalanine. (A) viable cell concentration of various cell pools without tyrosine and optionally supplemented with phenylalanine and (B) shows a graph of culture viability of the same pools.
**Fig. 7** shows a graph of growth characteristics of pre-adapted tyrosine prototrophic cell pools where phenylalanine supplementation has occurred prior to cell growth assessment. (A) viable cell concentration of cell pools and (B) shows a graph of culture viability of the same cell pools in the same conditions.
**Fig. 8** shows a graph of PAH mRNA amounts in various cell pools relative to control cells (top) and a graph of GCH1 mRNA amounts in various cell pools relative to control cells (bottom).
**Fig. 9** shows graphs of growth characteristics of co-expressing tyrosine and glutamine auxotrophic cell pools. (A) viable cell concentration (B) viability and (C) cell diameter.

### DETAILED DESCRIPTION OF THE INVENTION

### Definitions

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting. Headings, sub-headings or numbered or lettered elements, e.g., (a), (b), (i) etc., are presented merely for ease of reading. The use of headings or numbered or lettered elements in this document does not require the steps or elements be performed in alphabetical order or that the steps or elements are necessarily discrete from one another. Other features, objects, and advantages of the invention will be apparent from the description and drawings, and from the claims. It is also to be understood that the terminology used herein is for the purpose of describing particular embodiments only, and is not intended to be limiting.

"About" or "approximately" as the terms are used herein applied to one or more values of interest, refer to a value that is similar to a stated reference value. In certain embodiments, the term "approximately" or "about" refers to a range of values that fall within 5%, 4%, 3%, 2%, 1%, or less in either direction (greater than or less than) of the stated reference value unless otherwise stated or otherwise evident from the context (except where such number would exceed 100% of a possible value).

As used herein, the term "control element" refers to a nucleic acid suitable to regulate (e.g. increase or decrease) the expression of a coding sequence, e.g., a gene or sequence encoding a product or enzyme molecule. Control elements may comprise promoter sequences, enhancer sequences, or both promoter and enhancer sequences. Control elements may comprise continuous nucleic acid sequences, discontinuous nucleic acid sequences (sequences interrupted by other coding or non-coding nucleic acid sequences), or both. A single control element may be comprised on a single nucleic acid or more than one nucleic acid. In an embodiment, a control element may comprise sequences 5' or 3' of a coding sequence, e.g., the coding sequence of a recombinant, therapeutic, or repressor polypeptide. In an embodiment, a control element may comprise sequences within one or more introns of a gene, e.g., a gene encoding a recombinant, therapeutic, or repressor polypeptide. In an embodiment, a control element may be comprised, in part or in its entirety, within sequences 5' or 3' of a coding sequence, e.g., the coding sequence of a recombinant, therapeutic, or repressor polypeptide. In an embodiment, a control element may be comprised in part or in its entirety, within a coding sequence, e.g., the coding sequence of a recombinant, therapeutic, or repressor polypeptide. In an embodiment, a control element may be comprised in part or in its entirety, within one or more introns of a gene, e.g., a gene encoding a recombinant, therapeutic, or repressor polypeptide. In an embodiment, a single control element may comprise nucleic acid sequences i) proximal to (e.g., adjacent to or contained within) a gene, e.g., a gene encoding a recombinant, therapeutic, or repressor polypeptide, or ii) distal to (e.g., separated by 10 or more, 100 or more, 1000 or more, or 10,000 or more bases, or disposed on a distinct and separate nucleic acid) a gene, e.g., a gene encoding a recombinant, therapeutic, or repressor polypeptide.

The term "about" when referring to a measurable value such as an amount, a temporal duration, and the like, is meant to encompass variations of ±5%, or in some instances ±1%, or in some instances ±0.1% from the specified value, as such variations are appropriate to perform the disclosed methods.

The term 'bioreactor' used herein refers to an apparatus in which a biological reaction or process is undertaken. The processes may be undertaken at industrial, pilot and laboratory scales including micro- and nanoscales.

As used herein, the term "endogenous" refers to any material from or naturally produced inside an organism, cell, tissue or system.

As used herein, the term "exogenous" refers to any material introduced to or produced outside of an organism, cell, tissue or system. Accordingly, "exogenous nucleic acid" refers to a nucleic acid that is introduced to or produced outside of an organism, cell, tissue or system. In some embodiments, sequences of the exogenous nucleic acid are not naturally produced, or cannot be naturally found, inside the organism, cell, tissue, or system that the exogenous nucleic acid is introduced into. In some embodiments, the sequences of the exogenous nucleic acids are non-naturally occurring sequences, or encode non-naturally occurring products. In some embodiments, sequences of the exogenous nucleic acid can also be found in the organism, cell, tissue, or system that the exogenous nucleic acid is introduced into. For example, an exogenous nucleic acid may encode an enzyme under the control of a constitutively active promoter, where the cell the exogenous nucleic acid is introduced into contains an endogenous nucleic acid sequence encoding said enzyme (e.g., under the control of an endogenous promoter).

As used herein, the term "enzyme molecule" refers to a polypeptide having an enzymatic activity of interest. An enzyme molecule may share structural similarity (e.g., sequence homology) with a naturally occurring enzyme having the enzymatic activity of interest. In some instances, the enzyme molecule has at least 80% amino acid sequence identity (e.g., at least about 85%, 90%, 91%, 92%, 93%, 94%, 95%, 96%, 97%, 98%, 99%, or 100% sequence identity) to a naturally occurring enzyme having the enzymatic activity of interest. In some embodiments, the enzyme molecule is a variant of a naturally occurring enzyme (e.g., a variant comprising one or more amino acid sequence alterations (e.g., substitutions, deletions, or insertions) relative to the amino acid sequence of the naturally occurring enzyme). In some instances, the term "molecule," when used with an identifier for an enzyme (e.g., PAH or GCH1), refers to a polypeptide having the enzymatic activity of the identified enzyme. By way of example, the terms "PAH molecule" or "PAH enzyme molecule" as used herein, refer to a polypeptide having the enzymatic activity of PAH. By way of further example, the terms "GCH1 molecule" or "GCH1 enzyme molecule" as used herein refer to a polypeptide having the enzymatic activity of GCH1. In some embodiments, an enzyme molecule is or comprises a single polypeptide chain. In some embodiments, an enzyme molecule is or comprises a multi-polypeptide complex, e.g., an oligomer (e.g., a dimer, trimer, tetramer, pentamer, hexamer, octamer, decamer, or dodecamer).

As used herein, the term "enzymatically active fragment" refers to a portion of an enzyme or enzyme molecule that has the enzymatic activity of interest of the enzyme or enzyme molecule. In some embodiments, an enzymatically active fragment is a variant of an enzyme or enzyme molecule comprising a deletion (e.g., a truncation) relative to the enzyme or enzyme molecule. In some embodiments, the enzymatic activity of interest of the enzymatically active fragment is no more than 50, 40, 30, 20, or 10% reduced relative to the enzyme or enzyme molecule from which the enzymatically active fragment is derived.

As used herein, the terms "nucleic acid," "polynucleotide," or "nucleic acid molecule" are used interchangeably and refers to deoxyribonucleic acid (DNA) or ribonucleic acid (RNA), or a combination of a DNA or RNA thereof, and polymers thereof in either single- or double-stranded form. The term "nucleic acid" includes, but is not limited to, a gene, cDNA, or an RNA sequence (e.g., an mRNA). In one embodiment, the nucleic acid molecule is synthetic (e.g., chemically synthesized or artificial) or recombinant. Unless specifically limited, the term encompasses molecules containing analogues or derivatives of natural nucleotides that have similar binding properties as the reference nucleic acid and are metabolized in a manner similar to naturally or non-naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)). By "subject nucleic acid," as used herein, is meant any nucleic acid of interest, e.g., comprising a sequence encoding a product as described herein or a sequence encoding a production factor (e.g., a Lipid Metabolism Modifier (LMM), such as SCD1 and/or SREBF-1) as described herein, that may be desirably introduced into or present within a cell as described herein.

As used herein, the terms "peptide," "polypeptide," and "protein" are used interchangeably, and refer to a compound comprised of amino acid residues covalently linked by peptide bonds, or by means other than peptide bonds. A protein or peptide must contain at least two amino acids, and no limitation is placed on the maximum number of amino acids that can comprise a protein's or peptide's sequence. In one embodiment, a protein may comprise of more than one, e.g., two, three, four, five, or more, polypeptides, in which each polypeptide is associated to another by either covalent or non-covalent bonds/interactions. Polypeptides include any peptide or protein comprising two or more amino acids joined to each other by peptide bonds or by means other than peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others.

As used herein, the term "plurality" refers to more than one (e.g., two or more) of the grammatical object of the article. By way of example, "a plurality of cells" can mean two cells or more than two cells.

"Product" as that term is used herein refers to an entity, e.g., a compound (e.g., polypeptide (e.g., glycoprotein), nucleic acid, lipid, saccharide, polysaccharide, or any hybrid thereof), vesicle, exosome, or virus, that is produced, e.g., expressed, by a cell, e.g., a cell which has been modified or engineered to produce the product, e.g., a production cell. In some embodiments, the product is a protein or polypeptide product. In some embodiments, the product comprises a naturally occurring product. In some embodiments the product comprises a non-naturally occurring product. In some embodiments, a portion of the product is naturally occurring, while another portion of the product is non-naturally occurring. In some embodiments, the product is a polypeptide, e.g., a recombinant polypeptide. In some embodiments, the product is suitable for diagnostic or pre-clinical use. In some embodiments, the product is suitable for therapeutic use, e.g., for treatment of a disease. In some embodiments, a product is a recombinant or therapeutic protein described herein, e.g., in the section below entitled 'Polypeptides'. In some embodiments, a virus includes a naturally occurring virus, a recombinant virus, a recombinant viral particle, a virus-like particle (VLP), a viral vector, an inactivated (e.g., dead or incapable of infection) virus, a plurality of viral proteins, a viral capsid, or any fragment, subset of components, or variant thereof.

As used herein, a "production cell" refers to a cell capable of producing a product, e.g., a recombinant polypeptide. In some embodiments, a production cell comprises an exogenous nucleic acid encoding a product (e.g., recombinant polypeptide), e.g., operably linked to a control element that regulates expression of the product in the production cell. When cultured in appropriate conditions, e.g., conditions disclosed herein, e.g., in a bioreactor and appropriate media, the production cell produces, e.g., and secretes, product.

As used herein, a "production factor" refers to a polypeptide or nucleic acid that affects the properties of a production cell with respect to expression of recombinant products. For example the production factor may improve the quantity (e.g. specific productivity per cell or product titer) or product quality (e.g. correct folding and assembly, solubility and the like). The production factor may be for example a protein involved in lipid metabolism (e.g., a Lipid Metabolism Modifier, such as SCD1 and/or SREBF-1), protein synthesis, protein folding, posttranslational modifications, protein transport and/or protein secretion. It may also be a polypeptide or nucleic acid that inhibits the expression or activity of an endogenous protein. For example, the production factor may inhibit the expression of a non-essential endogenous protein that is highly expressed and secreted, to improve the production capacity of the cell.

As used herein, the term "promoter" refers to a sequence having sufficient sequences, e.g., from a naturally occurring or engineered promoter such that operably linking a coding sequence to the promoter results in the expression of the coding sequence. For example, a cytomegalovirus (CMV) promoter comprises all or an active fragment of the CMV promoter, e.g., all or an active fragment of the CMV promoter including optionally intron A and/or UTR sequences. In an embodiment, a CMV promoter differs at no more than 5, 10, 20, 30, 50, or 100 nucleotides from a naturally occurring or engineered variant CMV promoter. In an embodiment, a CMV promoter differs at no more than 1, 5, 10, or 50% of its nucleotides from a naturally occurring or engineered variant CMV promoter. Promoters, as used herein, may be constitutive, regulated, repressible, inducible, strong, weak, or other properties of the promoter sequences the promoters comprise. In an embodiment, a promoter may comprise sequences 5' or 3' of a coding sequence, e.g., the coding sequence of a recombinant, therapeutic, or repressor polypeptide. In an embodiment, a promoter may comprise sequences within one or more introns of a gene, e.g., a gene encoding a recombinant, therapeutic, or repressor polypeptide. In an embodiment, a promoter may be comprised, in part or in its entirety, within sequences 5' or 3' of a coding sequence, e.g., the coding sequence of a recombinant, therapeutic, or repressor polypeptide. In an embodiment, a promoter may be comprised in part or in its entirety, within a coding sequence, e.g., the coding sequence of a recombinant, therapeutic, or repressor polypeptide. In an embodiment, a promoter may be comprised in part or in its entirety, within one or more introns of a gene, e.g., a gene encoding a recombinant, therapeutic, or repressor polypeptide.

As used herein, the term "operably linked" refers to a relationship between a nucleic acid sequence encoding a product (e.g., a polypeptide) or enzyme molecule, and a control element, wherein the sequence encoding a product or enzyme molecule and the control element are operably linked if they are disposed in a manner suitable for the control element to regulate the expression of the sequence encoding a product or enzyme molecule. Thus for different control elements, operably linked will constitute different dispositions of the sequence encoding a product or enzyme molecule relative to the control element. For example, a sequence encoding a product (e.g., a polypeptide) may be operably linked to a control element comprising a promoter element if the promoter element and sequence encoding a product (e.g., a polypeptide) are disposed proximal to one another and on the same nucleic acid. In another example, a sequence encoding a product (e.g., a polypeptide) may be operably linked to a control element comprising an enhancer sequence that operates distally if the enhancer sequence and sequence encoding a product (e.g., a polypeptide) are disposed a suitable number of bases apart on the same nucleic acid, or even on distinct and separate nucleic acids.

As used herein, a selection marker refers to one or more nucleic acid sequences that confer a phenotype that may be used to select a cell comprising the one or more nucleic acid sequences. In some embodiments, the one or more nucleic acid sequences comprise a sequence encoding a polypeptide (e.g., and a suitable control element for expression of said polypeptide). For example, a selection marker may comprise a gene encoding a protein conferring an antibiotic resistance phenotype. Such a selection marker may be referred to as an antibiotic selection marker. In some embodiments, a selection marker comprises one or more nucleic acid sequences conveying the ability to survive (e.g., grow and divide during) a condition comprising a reduced level (e.g., the absence of) of an essential nutrient, e.g., a level insufficient for a cell to survive without the selection marker. For example, a selection marker may comprise a first nucleic acid encoding a PAH enzyme molecule and a second nucleic acid encoding a GCH1 enzyme molecule, wherein the selection marker conveys the ability to survive a reduced level (e.g., the absence of) tyrosine in the culture media. Such a selection marker may be referred to as an auxotrophy marker or auxotrophy selection marker. Appending a compound name, e.g., amino acid name, to auxotrophy marker or auxotrophy selection marker specifies the nutrient which the selection markers conveys the ability to survive a reduced level of or the absence of.

### Vectors and Vector Systems

The present invention uses vectors encoding components that enable transformed host cells to express a product of interest, such as a recombinant polypeptide, and to grow in low levels and the absence of tyrosine, which would otherwise be an essential amino acid for the cells and its absence would lead to cell death and/or poor to growth.

The vectors comprise three components. A first nucleic acid sequence which encodes a phenylalanine hydroxylase (PAH) enzyme molecule, which lacks a functional N-terminal regulatory domain; and a second nucleic acid sequences which encodes a GTP cyclohydrolase 1 (GCH1) enzyme molecule. These sequences are operably linked to control sequences which enable expression of the enzymes in a suitable host cell. In one embodiment, the control sequences include a CMV promoter or an SV40 promoter, for example a sequence encoding a human PAH sequence may be operably linked a control sequence comprising an SV40 promoter and/or a sequence encoding the GCH1 may be operably linked a control sequence comprising an SV40 promoter.

A third sequence comprises an insertion site into which a nucleic acid sequence encoding a product of interest can be cloned, for example a multiple cloning site. This site is positioned and operably lined to control sequences so that when the desired sequence has been introduced it can be expressed in a suitable host cell. In one embodiment, the three sequences, which can be considered as expression cassettes, are present in the same vector. In another embodiment, the first and second nucleic acid sequences could be on separate vectors provided that the third nucleic acid sequence is on the same vector as one of them to ensure selection of the sequence of interest is linked to the presence of a selectable marker.

The vectors may comprise additional expression cassettes for products of interest i.e. the vector system may comprise a fourth, and optionally a fifth and optionally a sixth nucleic acid sequence etc. each comprising an insertion site into which a nucleic acid sequence encoding a product of interest can be cloned, for example a multiple cloning site. As for the third nucleic acid sequence, these sites are positioned and operably lined to control sequences so that when the desired sequence has been introduced it can be expressed in a suitable host cell. Bispecific antibodies for example have at least three different and usually at least four different chains. These expression cassettes ready for insertion of sequences of interest can be configured in a variety of ways. If the PAH and GCH1 sequences are on different vectors then each vector may contain one or more expression cassettes with a multiple cloning site e.g. each may contain two such expression cassettes. In some embodiments the expression cassettes, each with a multiple cloning site may be present in a single vector with one of the selection markers only. Accordingly one vector may have three or four expression cassettes each with a multiple cloning site for introduction of the sequences of interest, such as the heavy or light chains for bispecific antibody production.

In one embodiment, and to take full advantage of the ability to introduce multiple sequences in the same step, all vector system components can be introduced into the host cell at the same time.

In another embodiment, a suitable host cell may already be engineered to comprise one of the first or second nucleic acid sequences.

The nucleic acid sequences encoding the recombinant product and PAH, GCH1 enzymes can be cloned into a number of types of vectors. For example, the nucleic acids can be cloned into a vector including, but not limited to a plasmid, a phagemid, a phage derivative, an animal virus, and a cosmid. Vectors of particular interest include expression vectors and replication vectors. In embodiments, the expression vector may be provided to a cell in the form of a viral vector. Viral vector technology is well known in the art and is described, for example, in Sambrook et al., 2012, MOLECULAR CLONING: A LABORATORY MANUAL, volumes 1-4, Cold Spring Harbor Press, NY), and in other virology and molecular biology manuals. Viruses, which are useful as vectors include, but are not limited to, retroviruses, adenoviruses, adeno- associated viruses, herpes viruses, and lentiviruses. In general, a suitable vector contains an origin of replication functional in at least one organism (and so the vectors may be self-replicating), a control element which comprises a promoter element and optionally an enhancer element, convenient restriction endonuclease sites, and one or more selectable markers, (e.g., WO 01/96584; WO 01/29058; and U.S. Pat. No. 6,326,193). Vectors derived from viruses are suitable tools to achieve long-term gene transfer since they allow long-term, stable integration of a transgene and its propagation in daughter cells.

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection, e.g., a selection marker or a reporter gene.

Vectors contemplated may comprise insertion sites suitable for inserting sequences encoding polypeptides, e.g., exogenous therapeutic polypeptides. Insertion sites may comprise restriction endonuclease sites.

Sequences encoding products of interest (as described in the section below entitled recombinant products) can be introduced into the vector system described here using cloning techniques well known in the art. The resulting vector system will then comprise in addition to the first and second nucleic acid sequences at least a third nucleic acid sequence comprising a sequence encoding a product of interest operably linked to a third control sequence which enables expression of the product in a host cell, which third sequence is present in the same vector as the first nucleic acid sequence and/or the second nucleic acid sequence (to ensure the selection markers function to select for cells that include third nucleic acid sequence).

As discussed above, the vector system of the present invention may be used to express multiple sequences of interest e.g. for proteins that have multiple subunits including antibodies (standard and bispecific antibodies). The vectors may therefore comprise additional expression cassettes for products of interest and multiple sequences of interest can be introduced into the multiple cloning sites to produce vectors ready to be introduced into host cells that can express a plurality of products of interest. Accordingly after introduction of the sequences of interest, in addition to the third nucleic sequence comprising a sequence encoding a product of interest operably linked to a third control sequence which enables expression of the product in a host cell, the vector system may comprise a fourth, and optionally a fifth and optionally a sixth nucleic acid sequence etc. each comprising a sequence encoding a product of interest operably linked to a control sequence which enables expression of the product in a host cell. These sequences will be present in the same vector as the first and/or second nucleic acid sequences (to ensure they are selected for as a result of being associated with a selectable marker).

Again as discussed above these expression cassettes can be configured in a variety of ways. If the PAH and GCH1 sequences are on different vectors then each vector may contain one or more expression cassettes each encoding a product of interest e.g. each may contain two such expression cassettes. In some embodiments the expression cassettes may be present in a single vector with one of the selection markers only. Accordingly one vector may have three or four expression cassettes each with a sequence encoding a product of interest, such as the heavy or light chains for bispecific antibody production.

The vectors may also contain sequences to assist with integration into the host cell genome either randomly or in a site-specific manner, such as the PiggyBac^{™} system that uses inverted terminal repeat sequences (ITRs) located on both ends of the vector. A sequence specific transposase which is included during the transfection process, site-specific integration methods and sequences are also described in WO2013/190032 and WO2018/150269.

In some embodiments, the vector comprising a nucleic acid sequence encoding a product comprises a further selection marker, as described below, such as glutamine synthetase. Typically the vector system includes a separate vector comprising a further selection marker, as described below, and a multiple cloning site for inserting one ore mores sequences encoding a product or products of interest operably linked to a control sequence which enables expression of the product in a host cell. Once the sequence of interest has been cloned into the multiple cloning site then the vector will comprise a further selection marker, as described below and a nucleic acid sequence comprising a sequence encoding a product of interest operably linked to a control sequence which enables expression of the product in a host cell. Such a vector generally does not include the PAH or GCH1 sequences.

The vector or vectors may be provided as a kit including instructions for use, and optionally transfection reagents and the like.

### GCH1 Enzyme Molecules

Naturally occurring GCH1 enzyme catalyzes the transformation of GTP into 7,8-dihydroneopterin 3'-triphosphate (consuming two water molecules and also producing acetic acid), the first step in the production of BH4. In some embodiments, a GCH1 enzyme molecule has the same or similar activity to a naturally occurring GCH1 enzyme. In some embodiments, a GCH1 enzyme molecule has increased or decreased activity relative naturally occurring GCH1 enzyme.

In some embodiments, a GCH1 enzyme molecule is a naturally occurring GCH1 enzyme. In some embodiments, a GCH1 enzyme molecule comprises a full-length (e.g., non-truncated) GCH1 enzyme. In some embodiments, the GCH1 molecule has at least 50% amino acid sequence identity (e.g., at least about 50%, 60%, 70%, 75%, 80%, 85%, 90%, 91%, 92%, 93%, 94%, 95%, 96%, 97%, 98%, 99%, or 100% sequence identity) to a mammalian GCH1 enzyme.

In some embodiments, a GCH1 enzyme molecule is a variant of a naturally occurring GCH1 enzyme or of a non-naturally occurring (e.g., synthetic) GCH1 enzyme (e.g., a variant comprising one or more amino acid sequence alterations (e.g., substitutions, deletions, or insertions) relative to the amino acid sequence of the naturally occurring or non-naturally occurring enzyme). In some embodiments, a GCH1 enzyme molecule is or comprises a deletion mutation, e.g., a truncation, e.g., a truncation of the N-terminal region, relative to a naturally occurring GCH1 enzyme. In some embodiments, a GCH1 enzyme molecule is or comprises at least 75, 80, 85, 90, 95, or 99% of the amino acid sequence of a naturally occurring GCH1 enzyme (and optionally, up to 100, 99, 95, 90, 85, 80, 79, 78, 77, 76, or 75% of the amino acid sequence). In some embodiments, a GCH1 enzyme molecule comprises no more than 99, 95, 90, 85, 84, 83, 82, 81, 80, 79, 78, 77, 76, or 75% of the amino acid sequence of a naturally occurring GCH1 enzyme.

In some embodiments, a GCH1 enzyme molecule is a monomer, e.g., is an active enzyme as a monomer. In some embodiments, a GCH1 enzyme molecule forms a multimer (e.g., under appropriate conditions for enzymatic activity, e.g., cellular or physiological conditions, e.g., during a biomanufacturing process), e.g., is an active enzyme as a multimer. In some embodiments, a GCH1 enzyme molecule multimer is a dimer, trimer, tetramer, pentamer, hexamer, heptamer, octamer, nonamer, or decamer, e.g., a decamer.

Sequences for use in GCH1 enzyme molecules of the present disclosure may be drawn from any known GCH1 enzyme sequences. In some embodiments, a GCH1 enzyme molecule comprises a human GCH1 enzyme, a variant thereof, or an enzymatically active fragment thereof. In some embodiments, a GCH1 enzyme molecule comprises a CHO GCH1 enzyme, a variant thereof, or an enzymatically active fragment thereof.

In some embodiments, a GCH1 enzyme molecule comprises the amino acid sequence encoded by SEQ ID NO: 1, e.g., the amino acid sequence of SEQ ID NO: 2. In some embodiments, a GCH1 enzyme molecule comprises the amino acid sequence encoded by NCBI Reference Sequence: NM_001024024 (e.g., as of 6 October 2019). In some embodiments, a GCH1 enzyme molecule comprises an amino acid sequence that is at least 80, 81, 82, 83, 84, 85, 86, 87, 88, 89, 90, 91, 91, 92, 93, 94, 95, 96, 97, 98, 99, or 100% identical to an amino acid sequence encoded by SEQ ID NO: 1, e.g., to the amino acid sequence of SEQ ID NO: 2. In some embodiments, an exogenous nucleic acid encoding a GCH1 enzyme molecule comprises a nucleic acid sequence that is at least 80, 81, 82, 83, 84, 85, 86, 87, 88, 89, 90, 91, 91, 92, 93, 94, 95, 96, 97, 98, 99, or 100% identical to the nucleic acid sequence of SEQ ID NO: 1.

### NCBI Reference Sequence: NM_001024024

ACGCGTATGGAGAAGGGCCCTGTGCGGGCACCGGCGGAGAAGCCGCGGGGCGCCAGGT
GCAGCAATGGGTTCCCCGAGCGGATCCGCCGCGGCCCGGGCCCAGCAGGCCGGCGGA
GAAGCCCCCGCGGCCCGAGGCCAAGAGCGCGCAGCCCGCGGACGGCTGGAAGGGCGA
GCGGCCCCGCAGCGAGGAGGATAACGAGCTGAACCTCCCTAACCTGGCAGCCGCCTACT
CGTCCATCCTGAGCTCGCTGGGCGAGAACCCCCAGCGGCAAGGGCTGCTCAAGACGCCC
TGGAGGGCGGCCTCGGCCATGCAGTTCTTCACCAAGGGCTACCAGGAGACCATCTCAGAT
GTCCTAAACGATGCTATATTTGATGAAGATCATGATGAGATGGTGATTGTGAAGGACATAG
ACATGTTTTCCATGTGTGAGCATCACTTGGTTCCATTTGTTGGAAAGGTCCATATTGGTTAT
CTTCCTAACAAGCAAGTCCTTGGCCTCAGCAAACTTGCGAGGATTGTAGAAATCTATAGTA
GAAGACTACAAGTTCAGGAGCGCCTTACAAAACAAATTGCTGTAGCAATCACGGAAGCCTT
GCGGCCTGCTGGAGTCGGGGTAGTGGTTGAAGCAACACACATGTGTATGGTAATGCGAGG

TGTACAGAAAATGAACAGCAAAACTGTGACCAGCACAATGTTGGGTGTGTTCCGGGAGGAT
CCAAAGACTCGGGAAGAGTTCCTGACTCTCATTAGGAGCTGACGTACGAACTTGTTTATTG
CAGCTTATAATGGTTACAAATAAAGCAATAGCATCACAAATTTCACAAATAAAGCATTTTTTT
CACTGCATTCTAGTTGTGGTTTGTCCAAACTCATCAATGTATCTTATCATGTCTGGATCGTC
GAC (SEQ ID NO: 1)

MEKGPVRAPAEKPRGARCSNGFPERDPPRPGPSRPAEKPPRPEAKSAQPADGWKGERPRS
EEDNELNLPNLAAAYSSILSSLGENPQRQGLLKTPWRAASAMQFFTKGYQETISDVLNDAIFDE
DHDEMVIVKDIDMFSMCEHHLVPFVGKVHIGYLPNKQVLGLSKLARIVEIYSRRLQVQERLTKQI
AVAITEALRPAGVGVVVEATHMCMVMRGVQKMNSKTVTSTMLGVFREDPKTREEFLTLIRS
(SEQ ID NO: 2)

### PAH Enzyme Molecules

Naturally occurring PAH enzyme catalyzes the transformation of phenylalanine to tyrosine using molecular oxygen and tetrahydrobiopterin (BH4).

In some embodiments, a PAH enzyme molecule is a variant of a naturally occurring PAH enzyme or of a non-naturally occurring (e.g., synthetic) PAH enzyme (e.g., a variant comprising one or more amino acid sequence alterations (e.g., substitutions, deletions, or insertions) relative to the amino acid sequence of the naturally occurring or non-naturally occurring enzyme). In some embodiments, a PAH enzyme molecule is or comprises a deletion mutation, e.g., a truncation, e.g., a truncation of the N-terminal region, relative to a naturally occurring PAH enzyme. In some embodiments, a PAH enzyme molecule is or comprises at least 75, 80, 85, 90, 95, or 99% of the amino acid sequence of a naturally occurring PAH enzyme (and optionally, up to 100, 99, 95, 90, 85, 80, 79, 78, 77, 76, or 75% of the amino acid sequence). In some embodiments, a PAH enzyme molecule comprises no more than 99, 95, 90, 85, 84, 83, 82, 81, 80, 79, 78, 77, 76, or 75% of the amino acid sequence of a naturally occurring PAH enzyme. In some embodiments, a PAH enzyme molecule is or comprises at least 200, 210, 220, 230, 240, 250, 260, 270, 280, 290, 300, 310, 320, 330, 335, or 336 amino acids of a naturally occurring PAH enzyme molecule (and optionally, no more than 450, 400, 390, 380, 370, 360, 350, 340, or 336 amino acids). In some embodiments, a PAH enzyme molecule is or comprises less than or equal to 450, 400, 390, 380, 370, 360, 350, 340, or 336 amino acids of a naturally occurring PAH enzyme molecule (and optionally, at least 200, 210, 220, 230, 240, 250, 260, 270, 280, 290, 300, 310, 320, 330, 335, or 336 amino acids). For example, a PAH enzyme molecule may comprise the first 1-14 and 37 and onward amino acids, comprising a deletion of amino acids 15-37. As a further example, a PAH enzyme molecule may comprise a deletion of amino acids 1-116. As a further example, a PAH enzyme molecule may comprise a deletion of amino acids 1-10 and 30-40. As a further example, a PAH enzyme molecule may comprise the 335, 336, 337, 338, 339, 340, 341, 342, 343, 344, 345, 346, 347, 348, 349, or 350 C-terminal amino acids of a naturally occurring PAH enzyme, e.g., the 343 C-terminal amino acids.

According to the invention, the PAH enzyme molecule lacks some or all of a regulatory domain of a naturally occurring PAH enzyme, such that the PAH enzyme molecule is constitutively active relative to the naturally occurring PAH enzyme. Without wishing to be bound by theory, PAH enzymes are understood to comprise an N-terminal region comprising one or more regulatory domains that regulate the enzymatic activity of PAH, e.g., by regulating access to the enzyme active site. The regulatory region may comprise an ACT domain, known to allow allosteric regulation of metabolic enzymes, and/or an active site lid that can conditionally block access to the enzyme active site. We believe that a PAH enzyme molecule lacking some or all of the regulatory domain is useful in a production cell, e.g., selection marker, described herein, because such a PAH enzyme molecule may be more active (e.g., constitutively active) than a PAH enzyme molecule comprising a full length PAH enzyme, e.g., a PAH enzyme molecule subject to the allosteric regulation of the regulatory domain. In some embodiments, a PAH enzyme molecule lacks an active site lid. In some embodiments, a PAH enzyme molecule lacks an ACT domain. In some embodiments, a PAH enzyme molecule comprises an alteration (e.g., a substitution, deletion, or insertion) that abolishes the regulatory (e.g., inhibitory) functions of the N-terminal regulatory region (e.g., the active site lid and/or ACT domain). In some embodiments, a PAH enzyme molecule is not appreciably inhibited (e.g., not inhibited) by the presence of phenylalanine. In some embodiments, the PAH enzyme molecule comprises a deletion of amino acids 1-10, 1-20, 1-30, 1-40, 1-50, 1-60, 1-70, 1-80, 1-90, 1-100, 1-110, or 1-116 (e.g., 1-116) or a deletion of residues corresponding to amino acids 1-10, 1-20, 1-30, 1-40, 1-50, 1-60, 1-70, 1-80, 1-90, 1-100, 1-110, or 1-116 (e.g., 1-116) of human PAH. In some embodiments, a PAH enzyme molecule lacks the N-terminal 116 amino acids of naturally occurring PAH enzyme (e.g., of naturally occurring human PAH enzyme) or the corresponding amino acids of a different naturally occurring PAH enzyme. See Daubner et al., 1997, Arch. Biochem. Biophys 348(2): 295 which describes a truncated PAH lacking the regulatory domain (first 116 amino acids). This truncated PAH expressed in *E.coli* was more stable, more soluble, did not require pre-incubation with phenylalanine to become active, and had a higher affinity for substrate). In some embodiments, a PAH enzyme molecule comprises the C-terminal region of a naturally occurring PAH enzyme, e.g., the catalytic and multimerization portions of the PAH enzyme.

In some embodiments, a PAH enzyme molecule is a monomer, e.g., is an active enzyme as a monomer. In some embodiments, a PAH enzyme molecule forms a multimer (e.g., under appropriate conditions for enzymatic activity, e.g., cellular or physiological conditions, e.g., during a biomanufacturing process), e.g., is an active enzyme as a multimer. In some embodiments, a PAH enzyme molecule multimer is a dimer, trimer, tetramer, pentamer, hexamer, heptamer, or octamer, e.g., a tetramer.

Sequences for use in PAH enzyme molecules of the present disclosure may be drawn from any known PAH enzyme sequences. In some embodiments, a PAH enzyme molecule comprises a human PAH enzyme, a variant thereof, or an enzymatically active fragment thereof. In some embodiments, the PAH molecule has at least 50% amino acid sequence identity (e.g., at least about 50%, 60%, 70%, 75%, 80%, 85%, 90%, 91%, 92%, 93%, 94%, 95%, 96%, 97%, 98%, 99%, or 100% sequence identity) to a human PAH enzyme. In some embodiments, a PAH enzyme molecule comprises a CHO PAH enzyme, a variant thereof, or an enzymatically active fragment thereof. In some instances, the PAH molecule has at least 50% amino acid sequence identity (e.g., at least about 50%, 60%, 70%, 75%, 80%, 85%, 90%, 91%, 92%, 93%, 94%, 95%, 96%, 97%, 98%, 99%, or 100% sequence identity) to a CHO PAH enzyme.

In some embodiments, a PAH enzyme molecule comprises the amino acid sequence encoded by any of SEQ ID NOs: 3 or 4, e.g., the amino acid sequence of any of SEQ ID NOs: 5 or 6. In some embodiments, a PAH enzyme molecule comprises an amino acid sequence that is at least 80, 81, 82, 83, 84, 85, 86, 87, 88, 89, 90, 91, 91, 92, 93, 94, 95, 96, 97, 98, 99, or 100% identical to an amino acid sequence encoded by any of SEQ ID NOs: 3 or 4, e.g., to the amino acid sequence of any of SEQ ID NOs: 5 or 6. In some embodiments, an exogenous nucleic acid encoding a PAH enzyme molecule comprises a nucleic acid sequence that is at least 80, 81, 82, 83, 84, 85, 86, 87, 88, 89, 90, 91, 91, 92, 93, 94, 95, 96, 97, 98, 99, or 100% identical to the nucleic acid sequence of any of SEQ ID NOs: 3 or 4.
Exemplary CHO PAH Nucleic Acid Sequence (NCBI Reference Sequence: XM_027434726.1) 
ATGGTGCCCTGGTTCCCAAGGACCATTCAAGAGCTGGACAGATTTGCCAATCAGATTCTCA
GTTATGGAGCAGAACTGGATGCAGACCACCCGGGCTTTAAAGATCCTGTGTACCGGGCGA
GGCGAAAGCAGTTTGCTGACATTGCCTACAACTACCGCCATGGGCAGCCCATCCCTCGGG
TGGAATACACAGAAGAAGAGAAGAAGACCTGGGGAACAGTGTTCAAGACACTGAAGGCCT
TGTATAAAACGCATGCCTGCTATGAACACAACCACATTTTCCCACTTCTGGAAAAGTACTGC
 
GGGTTCCGTGAAGACAACATTCCCCAGCTGGAAGATGTTTCTCAGTTTCTGCAGACTTGTA
CTGGTTTCCGCCTCCGACCTGTTGCTGGCTTACTGTCCTCTCGAGATTTCTTGGGTGGCCT
GGCCTTCCGAGTCTTCCACTGCACACAATACATCAGGCATGGGTCTAAGCCCATGTACACA
CCTGAACCAGACATTTGTCATGAACTGTTGGGACATGTGCCCTTGTTTTCAGATCGCAGCT
TTGCCCAGTTTTCCCAGGAAATCGGACTTGCTTCTCTGGGTGCACCTGACGAATACATCGA
GAAATTGGCCACAATTTACTGGTTTACTGTGGAGTTTGGGCTCTGCAAGGAAGGAGATTCC
ATCAAGGCATATGGTGCTGGGCTTCTGTCATCCTTTGGTGAATTACAGTACTGTTTATCAGA
CAAGCCGAAGCTCCTGCCCCTGGACCTAGAGAAGACAGCCTCACAGGAGTACAATGTCAC
AGAGTTCCAGCCCCTGTACTACGTGGCAGAGAGTTTCAATGATGCCAAGGAGAAAGTGAG
GGCCTTTGCTGCCACAATCCCCCGGCCCTTCTCGGTTCGCTATGATCCCTACACTCAAAGG
GTTGAGGTCCTGGACAACACTCAGCAGTTGAAGATTTTGGCTGACTCCATCAACAGTGAGG
TTGGAATCCTTTGCAGTGCCCTGCATAAAATAAAGTCATGA (SEQ ID NO: 3)
Exemplary CHO PAH Amino Acid Sequence 
MVPWFPRTIQELDRFANQILSYGAELDADHPGFKDPVYRARRKQFADIAYNYRHGQPIPRVEY
TEEEKKTWGTVFKTLKALYKTHACYEHNHIFPLLEKYCGFREDNIPQLEDVSQFLQTCTGFRLR
PVAGLLSSRDFLGGLAFRVFHCTQYIRHGSKPMYTPEPDICHELLGHVPLFSDRSFAQFSQEIG
LASLGAPDEYIEKLATIYWFTVEFGLCKEGDSIKAYGAGLLSSFGELQYCLSDKPKLLPLDLEKT
ASQEYNVTEFQPLYYVAESFNDAKEKVRAFAATIPRPFSVRYDPYTQRVEVLDNTQQLKILADSI
NSEVGILCSALHKIKS (SEQ ID NO: 5)
Exemplary Human PAH Nucleic acid sequence (GenBank: K03020.1) 
GCTAGCATGGTGCCCTGGTTCCCAAGAACCATTCAAGAGCTGGACAGATTTGCCAATCAGA
TTCTCAGCTATGGAGCGGAACTGGATGCTGACCACCCTGGTTTAAAGATCCTGTGTACCG
TGCAAGACGGAAGCAGTTTGCTGACATTGCCTACAACTACCGCCATGGGCAGCCCATCCC
TCGAGTGGAATACATGGAGGAAGAAAAGAAAACATGGGGCACAGTGTTCAAGACTCTGAA
GTCCTTGTATAAAACCCATGCTTGCTATGAGTACAATCACATTTTTCCACTTCTTGAAAAGTA
CTGTGGCTTCCATGAAGATAACATTCCCCAGCTGGAAGACGTTTCTCAATTCCTGCAGACT
TGCACTGGTTTCCGCCTCCGACCTGTGGCTGGCCTGCTTTCCTCTCGGGATTTCTTGGGT
GGCCTGGCCTTCCGAGTCTTCCACTGCACACAGTACATCAGACATGGATCCAAGCCCATG
TATACCCCCGAACCTGACATCTGCCATGAGCTGTTGGGACATGTGCCCTTGTTTTCAGATC
GCAGCTTTGCCCAGTTTTCCCAGGAAATTGGCCTTGCCTCTCTGGGTGCACCTGATGAATA
CATTGAAAAGCTCGCCACAATTTACTGGTTTACTGTGGAGTTTGGGCTCTGCAAACAAGGA
GACTCCATAAAGGCATATGGTGCTGGGCTCCTGTCATCCTTTGGTGAATTACAGTACTGCT
 
TATCAGAGAAGCCAAAGCTTCTCCCCCTGGAGCTGGAGAAGACAGCCATCCAAAATTACAC
TGTCACGGAGTTCCAGCCCCTGTATTACGTGGCAGAGAGTTTTAATGATGCCAAGGAGAAA
GTAAGGAACTTTGCTGCCACAATACCTCGGCCCTTCTCAGTTCGCTACGACCCATACACCC
AAAGGATTGAGGTCTTGGACAATACCCAGCAGCTTAAGATTTTGGCTGATTCCATTAACAGT
GAAATTGGAATCCTTTGCAGTGCCCTCCAGAAAATAAAGTAAAGATCT (SEQ ID NO: 4)
Exemplary Human PAH Amino acid sequence

MVPWFPRTIQELDRFANQILSYGAELDADHPGFKDPVYRARRKQFADIAYNYRHGQPIPRVEY
MEEEKKTWGTVFKTLKSLYKTHACYEYNHIFPLLEKYCGFHEDNIPQLEDVSQFLQTCTGFRLR
PVAGLLSSRDFLGGLAFRVFHCTQYIRHGSKPMYTPEPDICHELLGHVPLFSDRSFAQFSQEIG
LASLGAPDEYIEKLATIYWFTVEFGLCKQGDSIKAYGAGLLSSFGELQYCLSEKPKLLPLELEKT
AIQNYTVTEFQPLYYVAESFNDAKEKVRNFAATIPRPFSVRYDPYTQRIEVLDNTQQLKILADSIN
SEIGILCSALQKIK (SEQ ID NO: 6)

### Host Cells

The present disclosure is directed, in part, to host cells, comprising a tyrosine auxotrophy selection marker, e.g., a first nucleic acid which encodes a phenylalanine hydroxylase (PAH) enzyme molecule; and a second nucleic acid which encodes GTP cyclohydrolase 1 (GCH1) enzyme molecule. At least one of these sequences is exogenous to the host cell, i.e. not naturally present. Both sequences may be exogenous to the host cell.

As described in the section above relating to vectors, since the nucleic acid sequences can be in the same or different vectors, they may be present in the host cell in the same or different nucleic acid molecules/vectors. These vectors may be self-replicating vectors, particularly when maintained extrachromosomally. In some embodiments the first and/or second nucleic acid is/are integrated into the genome of the production cell.

The host cell following introduction of the vector system will also typically comprise a third exogenous nucleic acid sequence encoding a product of interest, these cells also being termed herein 'production cells'. The product is typically not naturally present in the unmodified host cell e.g. a biotherapeutic protein. The third nucleic sequence is present in the same nucleic acid as the first nucleic acid sequence and/or the second nucleic acid sequence, depending on how many vectors were used to produce the cell. In some embodiments, the third exogenous nucleic acid is integrated into the genome of the host cell. Additional exogenous nucleic acids may also be present that were introduced using the vector system of the present invention.

The first, second, and/or third etc. exogenous nucleic acids comprise one or more control elements. A control element, e.g., a promoter and/or enhancer, is operably linked to the sequence encoding the PAH enzyme molecule, the sequence encoding the GCH1 molecule, or a sequence encoding a product. Control elements suitable for use in the present invention are known to those of skill in the art, and examples of which are also described herein.

### Host Cell Types

Generally, the methods herein can be used to produce a host cell, e.g., a cell or cell line comprising a nucleic acid construct (e.g., a vector or a heterologous nucleic acid integrated into the genome) comprising (i) a subject nucleic acid sequence encoding a product of interest and (ii) one or more exogenous nucleic acid sequence(s) encoding one or more enzyme molecule(s) that participate in the biosynthetic pathway of an amino acid, wherein the cell or cell line does not endogenously express the enzyme molecule(s).

Typically the cell is one suitable for large scale culture to produce a product of interest.

The host cell prior to introduction of the vector system of the present invention is unable to produce sufficient levels of tyrosine to support cell growth in the absence of tyrosine. This may because it is naturally does not express to sufficient levels one or more of the necessary enzymes for tyrosine biosynthesis or it has been engineered to knock out the relevant genes. Thus, in one embodiment a host cell of the present invention has been genetically modified to inhibit or abolish any endogenous PAH and/or GCH1 activity. This can for example be achieved by mutations (insertions, deletions and/or substitutions) in the genomic sequences encoding and/or regulating expression of endogenous PAH and/or GCH1.

The host cell is a eukaryotic cell, for example a mammalian, yeast or insect cell.

In one embodiment, the host cell is a mammalian cell. Example species from which host cell can be derived include human, mouse, rat, Chinese hamster, Syrian hamster, monkey, ape, dog, horse, ferret, and cat.

In embodiments, the host cell is a Chinese hamster ovary (CHO) cell. In one embodiment, the host cell is a CHO-K1 cell, a CHOK1SV^{®} cell, a DG44 CHO cell, a DUXB11 CHO cell, a CHO-S, a CHO GS knock-out cell (a CHO cell where all endogenous copies of the glutathione synthetase (GS) gene have been inactivated), a CHOK1SV^{®} FUT8 knock-out cell, a CHOZN, or a CHO-derived cell. The CHO GS knock-out cell (e.g., GS-KO cell) is, for example, a CHOK1SV^{®} GS knockout cell (such as a GS Xceed^{®} cell - CHOK1SV GS-KO^{®}, Lonza Biologics, Inc.). The CHO FUT8 knockout cell is, for example, the Potelligent^{®} CHOK1SV^{®} FUT8 knock-out (Lonza Biologics, Inc.).

In embodiments, the host cell is a HeLa, MDCK, Sf9, Sf21, Tn5, HT1080, NB324K, FLYRD18, HEK293, HEK293T, HT1080, H9, HepG2, MCF7, Jurkat, NIH3T3, PC12, PER.C6, BHK (baby hamster kidney), VERO, SP2/0, NS0, YB2/0, Y0, EB66, C127, L cell, COS (e.g., COS1 and COS7), QC1-3, CHOK1, CHOK1SV, Potelligent^{®} (CHOK1SV FUT8-KO), CHO GS knockout, GS Xceed^{™} (CHOK1SV GS-KO), CHOS, CHO DG44, CHO DXB11, or CHOZN cell, or any cells derived therefrom.

In other embodiments, the host cell is a cell other than a mammalian cell, such as avian, fish, insect, plant, fungus, or yeast cell.

In some embodiments, the host cell or the host cell's cell line was formed by a process comprising the fusion of a plurality of cells (e.g., the fusion of two cells of the same type (e.g., two CHO cells) or of different types (e.g., of different species)). Examples of host cells or cell lines formed by a process comprising the fusion of a plurality of cells include, but are not limited to, hybridomas, triomas, and quadromas.

In some embodiments, derived therefrom includes but is not limited to a cell described herein further comprising an alteration (e.g., knock-in of a gene, knock-out of a gene, or multiplicity of a gene) such as a mutation (e.g., substitution, deletion, or insertion) or the addition of a nucleic acid (e.g., a vector). In some embodiments, derived therefrom includes a cell described herein subjected to directed evolution. In some embodiments, derived therefrom includes a combination of these exemplary modifications described herein.

Eukaryotic cells include stem cells. The stem cells can be, for example, pluripotent stem cells, including embryonic stem cells (ESCs), adult stem cells, induced pluripotent stem cells (iPSCs), tissue specific stem cells (e.g., hematopoietic stem cells) and mesenchymal stem cells (MSCs).

In embodiments, the host cell is a differentiated form of any of the cells described herein. In one embodiment, the host cell is a cell derived from any primary cell in culture.

In embodiments, the host cell is a hepatocyte such as a human hepatocyte, animal hepatocyte, or a non-parenchymal cell. For example, the host cell can be a plateable metabolism qualified human hepatocyte, a plateable induction qualified human hepatocyte, plateable Qualyst Transporter Certified^{™} human hepatocyte, suspension qualified human hepatocyte (including 10-donor and 20-donor pooled hepatocytes), human hepatic Küpffer cells, human hepatic stellate cells, dog hepatocytes (including single and pooled Beagle hepatocytes), mouse hepatocytes (including CD-1 and C57BI/6 hepatocytes), rat hepatocytes (including Sprague-Dawley, Wistar Han, and Wistar hepatocytes), monkey hepatocytes (including Cynomolgus or Rhesus monkey hepatocytes), cat hepatocytes (including Domestic Shorthair hepatocytes), and rabbit hepatocytes (including New Zealand White hepatocytes). Exemplary hepatocytes are commercially available from Triangle Research Labs, LLC, 6 Davis Drive Research Triangle Park, North Carolina, USA 27709.

In some embodiments, the host cell comprises a knockout of glutamine synthetase (GS). In embodiments, the host cell does not comprise a functional GS gene. In embodiments, the host cell does not comprise a GS gene. In embodiments, the GS gene in a host cell comprises a mutation that renders the gene incapable of encoding a functional GS protein.

In embodiments, the eukaryotic cell is a lower eukaryotic cell such as e.g. a yeast cell (e.g., *Pichia* genus (e.g. *Pichia pastoris, Pichia methanolica, Pichia kluyveri,* and *Pichia angusta*), *Komagataella* genus (e.g. *Komagataella pastoris, Komagataella pseudopastoris* or *Komagataella phaffii*), *Saccharomyces* genus (e.g. *Saccharomyces cerevisae, cerevisiae, Saccharomyces kluyveri, Saccharomyces uvarum*), *Kluyveromyces* genus (e.g. *Kluyveromyces lactis, Kluyveromyces marxianus*), the *Candida* genus (e.g. *Candida utilis, Candida cacaoi, Candida boidinii,*)*,* the *Geotrichum* genus (e.g. *Geotrichum fermentans*), *Hansenula polymorpha, Yarrowia lipolytica,* or *Schizosaccharomyces pombe.* In some embodiments, the eukaryotic cell is of the species *Pichia pastoris.* Examples for *Pichia pastoris* strains include but are not limited to X33, GS115, KM71, KM71H, and CBS7435.

In embodiments, the eukaryotic cell is a fungal cell (e.g. *Aspergillus sp.* (such as *A*. *niger, A. fumigatus, A. orzyae, A. nidula), Acremonium sp.* (such as *A. thermophilum*), *Chaetomium sp.* (such as *C. thermophilum*), *Chrysosporium sp.* (such as *C. thermophile*), *Cordyceps sp.* (such as *C. militaris*), *Corynascus sp., Ctenomyces sp., Fusarium sp.* (such as *F. oxysporum*), *Glomerella* sp. (such as *G. graminicola*), *Hypocrea sp.* (such as *H. jecorina*), *Magnaporthe sp.* (such as *M. orzyae*)*, Myceliophthora sp.* (such as *M. thermophile*), *Nectria sp.* (such as *N. heamatococca*)*, Neurospora sp.* (such as *N. crassa*), *Penicillium sp., Sporotrichum sp.* (such as *S. thermophile*), *Thielavia sp.* (such as *T. terrestris, T. heterothallica*), *Trichoderma sp.* (such as *T. reesei*), or *Verticillium sp.* (such as *V. dahlia*))*.*

In embodiments, the eukaryotic cell is an insect cell (e.g., Sf9, Mimic^{™} Sf9, Sf21, High Five^{™} (BT1-TN-5B1-4), or BT1-Ea88 cells), an algae cell (e.g., of the genus *Amphora sp., Bacillariophyceae sp., Dunaliella sp., Chlorella sp., Chlamydomonas sp., Cyanophyta sp.* (cyanobacteria), *Nannochloropsis sp., Spirulina sp.,or Ochromonas sp.*), or a plant cell (e.g., cells from monocotyledonous plants (e.g., maize, rice, wheat, or *Setaria sp.*)*,* or from a dicotyledonous plants (e.g., cassava, potato, soybean, tomato, tobacco, alfalfa, *Physcomitrella patens* or *Arabidopsis sp.*)*.*

Suitable host cells are commercially available, for example, from culture collections such as the DSMZ (Deutsche Sammlung von Mikroorganismen and Zellkulturen GmbH, Braunschweig, Germany) or the American Type Culture Collection (ATCC).

### Additional Selection Markers

In some embodiments, a host cell comprises one or more selection markers in addition to the tyrosine auxotrophy selection marker. In some embodiments, the second selection marker is a different auxotrophy selection marker, such as a different amino acid auxotrophy selection marker. In one embodiment, the amino acid is proline or glutamine. Examples of nucleic acid sequences needed for such a selection marker are sequences encoding glutamine synthetase (for glutamine) and pyrroline-5-carboxylate synthase (P5CS) (for proline).

Another selection marker is dihydrofolate reductase (DHFR), e.g., an exogenous nucleic acid encoding a DHFR enzyme molecule, e.g., which confers resistance to methotrexate (MTX). In some embodiments, a DHFR selection marker is also a thymidine auxotrophy selection marker and/or a hypoxanthine auxotrophy selection marker. In some embodiments, a host cell does not comprise an endogenous functional DHFR gene, e.g., comprises a mutation that renders the endogenous DHFR gene incapable of encoding a functional DHFR enzyme.

A further selection marker comprises a hypoxanthine-guanine phosphoribosyltransferase (HPRT) selection marker, e.g., an exogenous nucleic acid encoding an HPRT enzyme molecule. In some embodiments, a production cell cannot grow and/or divide in the presence of aminopterin without HPRT (e.g., supplemental HPRT encoded by an exogenous nucleic acid) and a supplemental purine, e.g., hypoxanthine. In some embodiments, a HPRT selection marker is also a purine (e.g., hypoxanthine or guanine) auxotrophy selection marker. In some embodiments, a production cell does not comprise an endogenous functional HPRT gene, e.g., comprises a mutation that renders the endogenous HPRT gene incapable of encoding a functional HPRT enzyme.

In one embodiment, the selection marker is compatible with the Selexis selection system (e.g., SUREtechnology Platform ^{™} and Selexis Genetic Elements^{™}, commercially available from Selexis SA) or the Catalent GPEx^{®} selection system.

A selection marker for use in a production cell may be associated with a subject nucleic acid. Associated with, as used herein in reference to a relationship between a selection marker and a subject nucleic acid, refers to a relationship where the presence of the selection marker in a production cell correlates with the presence of the subject nucleic acid. A selection marker is associated with a subject nucleic acid such that selecting for (e.g., requiring) the presence of the selection marker in a production cell selects for the presence of the subject nucleic acid. In some embodiments, a selection marker, e.g., at least one component of a selection marker, is situated on the same nucleic acid molecule as a subject nucleic acid, e.g., on the same vector as a subject nucleic acid. For example, a production cell comprising a tyrosine auxotrophy selection marker comprising a first exogenous nucleic acid which encodes a PAH enzyme molecule and a second exogenous nucleic acid which encodes a GCH1 enzyme molecule may comprise a subject nucleic acid situated on the same vector as either the first exogenous nucleic acid or the second exogenous nucleic acid. In production cells comprising more than one selection marker, each selection marker may be associated with a different subject nucleic acid. In some embodiments, a production cell comprises a first selection marker associated with a first subject nucleic acid (e.g., encoding a product) and a second selection marker associated with second subject nucleic acid (e.g., encoding a production factor, e.g., a lipid metabolism modulator (LMM) such as SCD1 and/or SREBF-1 as described in WO2017/191165 and WO2019/152876). Thus the further selection marker is used to maintain the exogenous production factor that has been introduced in the host cell (including on a previous occasion to produce a stable cell line). In some embodiments, a production cell comprises a first selection marker associated with a first subject nucleic acid (e.g., encoding a first product) and a second selection marker associated with second subject nucleic acid (e.g., encoding a second product). In some embodiments, a production cell comprises a first selection marker associated with a first subject nucleic acid (e.g., encoding a first polypeptide of a multi-polypeptide product) and a second selection marker associated with second subject nucleic acid (e.g., encoding a second polypeptide of a multi-polypeptide product). It will be understood that additional subject nucleic acids can be included which may be associated with different markers or the same markers.

### Inhibitors

A host cell and/or culture comprising a host cell may comprise one or more enzyme molecule inhibitors (also referred to herein as an inhibitor). An enzyme molecule inhibitor can be used to increase the stringency of the selection processes described herein by reducing or preventing endogenous enzyme molecule activity, e.g., such that cells that do not take up the exogenous nucleic acid encoding the enzyme molecule (e.g., and comprising the subject nucleic acid sequence) exhibit reduced or undetectable levels of endogenous enzyme molecule activity. Cells exhibiting reduced or undetectable levels of endogenous enzyme molecule activity may not be able to grow and/or survive in the absence of an external supply of the amino acid (e.g., proline, tyrosine or glutamine) for which synthesis requires the activity of the enzyme molecule. In some embodiments, the inhibitor binds to the enzyme molecule, e.g., it binds to and inhibits the enzyme molecule. In embodiments, the inhibitor is an allosteric inhibitor of the enzyme molecule. In embodiments, the inhibitor is a competitive inhibitor of the enzyme molecule.

The production cells described herein may, in some embodiments, further comprise an inhibitor of an enzyme molecule that is being expressed by an exogenous nucleic acid introduced into the cell (e.g., PAH or GCH1). In some embodiments, the level of inhibitor in the cell is sufficient to reduce endogenous enzyme molecule activity to less than about 0.001%, 0.01%, 0.1%, 1%, 2%, 3%, 4%, 5%, or 10% of that observed in a cell lacking the inhibitor. In some embodiments, less than about 0.001%, 0.01%, 0.1%, 1%, 5%, or 10% of cells selected on the basis of growth in media lacking the amino acid do not comprise the subject nucleic acid. In some embodiments, the ratio of enzyme molecules and inhibitor molecules in the cell is about 1:1000, 1:500, 1:250, 1:200, 1:100, 1:90, 1:80, 1:70, 1:60, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 15:1, 20:1, 30:1, 40:1, 50:1, 60:1, 70:1, 80:1, 90:1, 100:1, 200:1, 250:1, 500:1, or 1000:1.

The inhibitor can be, for example, an amino acid or analog thereof, a polypeptide, a nucleic acid, or a small molecule. In some embodiments, the inhibitor is an analog of the amino acid produced by the biosynthetic pathway in which the enzyme molecule participates. In some embodiments, the inhibitor is an analog of a substrate of the enzyme molecule. In some embodiments, the inhibitor is an antibody molecule (e.g., an antibody or an antibody fragment, e.g., as described herein), a fusion protein, a hormone, a cytokine, a growth factor, an enzyme, a glycoprotein, a lipoprotein, a reporter protein, a therapeutic peptide, an aptamer, or a structural and/or functional fragment or hybrid of any of these. In some embodiments, the inhibitor is an antisense RNA, siRNA, tRNA, ribosomal RNA, microRNA, piRNA, snoRNA, snRNA, exRNA, scaRNA, an RNA aptamer, or long noncoding RNA.

In some embodiments, the inhibitor inhibits an enzyme molecule in the biosynthesis pathway for proline, tyrosine, or glutamine. In one embodiments, the inhibitor inhibits the activity of PAH (for example a phenylalanine analog) or GCH1. In embodiments, the inhibitor is a tetrahydrobiopterin (BH4) analog. In some embodiments, the inhibitor is a GTP analog. In one embodiment the inhibitor is selected from α-methyl tyrosine (e.g., at 50-100 µM), α-methyl phenylalanine and 2,4-amino-6-hydroxy pyrimidine.

In embodiments, the inhibitor inhibits the activity of an enzyme that forms the basis of one the additional selection markers, where used, such as a pyrroline-5-carboxylate synthase (P5CS) molecule. In embodiments, the inhibitor inhibits the activity of P5CS. In embodiments, the inhibitor is a proline analog. In embodiments, the inhibitor is L-azetidine-2-carboxylic acid, 3,4-dehydro-L-proline, or L-4-thiazolidinecarboxylic acid. In some embodiments, the inhibitor inhibits the activity of DHFR, e.g., is methotrexate. In some embodiments, the inhibitor inhibits glutamine synthetase, e.g., a glutamine analog, methionine sulphoximine (MSX) or an analog thereof (e.g., alpha-methyl or alpha-ethyl MSX). In some embodiments, a production cell comprises more than one selection marker and comprises an enzyme molecule inhibitor for each selection marker.

### Introduction of nucleic acids into host cells and selection steps

Many suitable methods are known in the art for introducing exogenous nucleic acids into a host cell and include, for example, transfection, transduction (e.g., viral transduction), or electroporation, e.g., of a nucleic acid, e.g., a vector, into the cell. Examples of physical methods for introducing a nucleic acid, e.g., a heterologous nucleic acid or vector described herein, into a host cell include, without limitation, calcium phosphate precipitation, particle bombardment, microinjection, electroporation, and the like. Methods for producing cells comprising vectors and/or exogenous nucleic acids are well-known in the art. See, for example, Sambrook et al., 2012, MOLECULAR CLONING: A LABORATORY MANUAL, volumes 1-4, Cold Spring Harbor Press, NY). Examples of chemical means for introducing a nucleic acid, e.g., a heterologous nucleic acid or vector described herein, into a host cell include, without limitation, lipofection, colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle). Other methods of state-of-the-art targeted delivery of nucleic acids are available, such as delivery of polynucleotides with targeted nanoparticles or other suitable sub-micron sized delivery system.

Host cells may be transiently transfected with the nucleic acids or stably transfected.

Selection of host cells that contain the introduced nucleic acids can be achieved by culturing the cells under stringent selection conditions that permit cells containing the introduced nucleic acids to grow whilst limiting the ability of non-transformed cells to grow, based on the tyrosine auxotrophy selection system of the invention (and any other additional selection markers that may have been included).

The population of transfected/transformed cells is cultured under conditions where the levels of tyrosine readily permit the selection of cells containing the introduced nucleic acids. Thus the cells are cultured in the presence of a level of tyrosine lower than the level required for survival or growth of a cell conditions. Typically, this will involve the use of media that lack tyrosine so that the cells are cultured in the absence of tyrosine. Nonetheless, low levels of tyrosine may be permitted as long as the selection conditions are sufficiently stringent, such as culture media comprising less than 0.01 g/L tyrosine, or less than 50, 20, or 10 µM tyrosine. A person skilled in the art will readily be able to determine the desired tyrosine levels to obtain a satisfactory selection stringency.

Since the enzyme molecule(s) supplied by the one or more exogenous nucleic acids provide activity that transforms phenylalanine into tyrosine it may be desirable to supplement the culture medium with additional phenylalanine so that host cells normal requirements for phenylalanine are met, as well as the provision of precursor for tyrosine production. Thus the population of cells can be cultured in the presence of a level of phenylalanine that is higher than the level required for survival or growth of a production cell cultured in the presence of the level of tyrosine required for survival or growth. Accordingly, in some embodiments, phenylalanine is provided (e.g., as part of culturing and/or as a component of the culture media) at a level of at least 0.035 g/L . The cells can therefore be cultured in the presence of a level of phenylalanine that is at least 2, 3 or 4 mM. Since high levels of phenylalanine can be inhibitory to cell growth, typically the level of phenylalanine is less than 10 mM, such as less than 9, 8, 7 or 6 mM.

In the case of the CHO PAH enzyme, in one embodiment it is preferred that the phenylalanine levels in the culture medium are from 2 to 9 mM, such as from 2 or 3 mM to 6 or 7 mM phenylalanine whereas in the case of the human PAH enzyme in one embodiment a preferred range is from 4 to 9 mM phenylalanine.

The cells may be subject to an adaption step so they can adjust to higher levels of phenylalanine. This step may involving passaging the cells at one or more increasingly higher concentrations of phenylalanine in the cell culture medium, such as 3 mM for one or two passages and then a final desired concentration, for example 6 mM. This may be performed prior to transfection or after transfection, for example as the cells are recovering ahead of a growth phase.

In some embodiments, the level of the phenylalanine is established and/or maintained using an auto-adjusting system that detects and/or monitors the level of phenylalanine in the culture and, responsive to the detected level being less than a threshold value, provides the phenylalanine (e.g., until the detected level is greater than or equal to the threshold value). In some embodiments, such an auto-adjusting system utilizes spectroscopy (e.g., Raman spectroscopy) to detect and/or monitor the level of the phenylalanine. Similar considerations apply where an additional selection marker is used.

Where two selection markers are used e.g. the selection system of the present invention and a GS selection system, the relevant vectors may be introduced at the same time and the cell culture medium formulated to provide stringent selection for both types of marker, i.e. medium lacking tyrosine and glutamine, optionally supplemented with phenylalanine, for example as described above. Alternatively, the selection may be a two-step process whereby one vector system is introduced and selected for under stringent conditions for the first marker, and then resulting selecting cells transfected/transformed under stringent conditions for the second marker, and optionally for the first marker, for example medium lacking tyrosine and glutamine, optionally supplemented with phenylalanine. Alternatively, less stringent conditions may be used for the first marker when selecting subsequently for the second marker. Culture conditions described above apply *mutatis mutandis* to this two selection marker procedure (and if additional markers are used).

### Functional characteristics of production cells containing introduced nucleic acid sequence

In some embodiments, a host cell comprising the tyrosine auxotrophy selection marker (a first exogenous nucleic acid which encodes a PAH enzyme molecule which lacks a functional N-terminal regulatory domain such that the PAH enzyme molecule is constitutively active and a second exogenous nucleic acid which encodes a GCH1 enzyme molecule) is able to grow and/or divide in culture media comprising a reduced level of tyrosine (e.g., in the absence of tyrosine). Such cells are also termed herein as production cells. The ability to grow and/or divide may be assessed by methods known to those of skill in the art and described herein. In some embodiments, a host cell is able to grow and/or divide in culture media comprising less than 0.01 g/L tyrosine, or less than 50, 20, or 10 µM tyrosine, e.g., in the absence of tyrosine. In some embodiments, a host cell is able to grow and/or divide in culture media that lacks tyrosine.

In some embodiments, a host cell comprises at least a threshold number of copies of a subject nucleic acid (e.g., a number of copies that is sufficient to efficiently produce a product), e.g., at least 1, 2, 5, 10, 20, 30, 40, 50, 60, 70, 80, 90, 100, 200, 300, 400, or 500 copies of the subject nucleic acid.

In some embodiments, a host cell comprises at least a threshold number of copies of the first exogenous nucleic acid (e.g., a number of copies that is sufficient to allow the host cell to grow and/or divide at a reduced level of (e.g., in the absence of tyrosine), e.g., at least 1, 2, 5, 10, 20, 30, 40, 50, 60, 70, 80, 90, 100, 200, 300, 400, or 500 copies of the first exogenous nucleic acid. In some embodiments, a host cell comprises at least a threshold number of copies of the second exogenous nucleic acid (e.g., a number of copies that is sufficient to allow the host cell to grow and/or divide at a reduced level of (e.g., in the absence of) tyrosine), e.g., at least 1, 2, 5, 10, 20, 30, 40, 50, 60, 70, 80, 90, 100, 200, 300, 400, 500, 1000, or 10,000 copies of the second exogenous nucleic acid.

In some embodiments, the subject nucleic acid, e.g., due to its association with the selection marker, persists in a host cell (e.g., or its daughter cells or descendants) over a designated interval. In some embodiments, the first exogenous nucleic acid persists in a host cell (e.g., or its daughter cells, descendants, generations, or population doublings) for at least 1, 2, 3, 4, 5, 6, 7, 8, 9, 10, 11, 12, 13, or 14 days, or at least 1, 2, 3, 4, 5, 6, 7, 8, 9, 10, 11, or 12 months (and optionally persists indefinitely). In some embodiments, the first exogenous nucleic acid persists in a host cell (e.g., or its daughter cells or descendants) for at least 1, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, 200, 220, 240, 260, 280, or 300 cell divisions (and optionally persists indefinitely). In some embodiments, the first exogenous nucleic acid persists in a host cell (e.g., or its daughter cells, descendants, generations, or population doublings) for at least 1, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, 200, 220, 240, 260, 280, or 300 host cycles, e.g., of a bioreactor described herein, population doublings, or number of generations, (and optionally persists indefinitely). In some embodiments, the second exogenous nucleic acid persists in a host cell (e.g., or its daughter cells, descendants, generations, or population doublings) for at least 1, 2, 3, 4, 5, 6, 7, 8, 9, 10, 11, 12, 13, or 14 days, or at least 1, 2, 3, 4, 5, 6, 7, 8, 9, 10, 11, or 12 months (and optionally persists indefinitely). In some embodiments, the second exogenous nucleic acid persists in a host cell (e.g., or its daughter cells, descendants, generations, or population doublings) for at least 1, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, 200, 220, 240, 260, 280, or 300 cell divisions (and optionally persists indefinitely). In some embodiments, the second exogenous nucleic acid persists in a host cell (e.g., or its daughter cells, descendants, generations, or population doublings) for at least 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, or 150 host cycles, e.g., of a bioreactor described herein, population doublings, or number of generations, (and optionally persists indefinitely). In some embodiments, the first exogenous nucleic acid persists in a host cell (e.g., or its daughter cells, descendants, generations, or population doublings) for as long as the host cell is maintained in media comprising a reduced level of tyrosine (e.g., not comprising tyrosine). In some embodiments, the second exogenous nucleic acid persists in a host cell (e.g., or its daughter cells, descendants, generations, or population doublings) for as long as the host cell is maintained in media comprising a reduced level of tyrosine (e.g., not comprising tyrosine). In some embodiments, persistence of the first, second, or first and second exogenous nucleic acids in a host cell is evaluated functionally, e.g., by whether the host cell grows and produces product in media comprising a reduced level of tyrosine (e.g., not comprising tyrosine). In some embodiments, persistence of the first, second, or first and second exogenous nucleic acids in a host cell is evaluated (e.g., confirmed) by using RT-PCR.

In some embodiments, a host cell comprising the tyrosine auxotrophy selection marker grows and/or divides faster than an otherwise similar cell that does not comprise the tyrosine auxotrophy selection marker in culture media comprising a reduced level of tyrosine (e.g., in the absence of tyrosine). In some embodiments, a host cell grows and/or divides at least 10, 20, 30, 40, 50, 60, 70, 80, 90, or 100% faster, or 10 times, 10² times, 10³ times, 10⁴ times, 10⁵ times, or 10⁶ times faster than a similar cell that does not comprise the tyrosine auxotrophy selection marker in culture media comprising a reduced level of tyrosine (e.g., in the absence of tyrosine).

In some embodiments, a host cell comprising the selection marker comprising an exogenous nucleic acid encoding an enzyme molecule (and optionally a subject nucleic acid associated with said exogenous nucleic acid) exhibits elevated enzyme molecule activity compared to cells lacking the exogenous nucleic acid and/or subject nucleic acid. In some embodiments, the level of enzyme molecule activity is increased by at least 10%, 20%, 30%, 40%, 50%, 60%, 70%, 80%, 90%, 100%, 200%, 500%, 1000%, or more relative to enzyme molecule activity detectable in cells lacking the exogenous nucleic acid encoding the enzyme molecule and/or the associated subject nucleic acid. In some embodiments, cells with the elevated activity may grow more quickly than cells lacking the exogenous nucleic acid encoding the enzyme molecule and/or the associated subject nucleic acid. In some embodiments, the rate of cell growth and/or division is increased by at least 10%, 20%, 30%, 40%, 50%, 60%, 70%, 80%, 90%, 100%, 200%, 500%, 1000%, or more relative to a similar cell in similar media conditions that lacks the exogenous nucleic acid encoding the enzyme molecule and/or the associated subject nucleic acid. In some embodiments, the host cell grows at least about 1.5, 2, 3, 4, 5, 6, 7, 8, 9, 10, 20, 30, 40, 50, 60, 70, 80, 90, 100, 150, 200, 250, 300, 400, 500, 600, 700, 800, 900, 1000, 1500, 2000, 5000, or 10,000 times more quickly on media lacking the amino acid than a similar cell lacking the subject nucleic acid and/or exogenous nucleic acid encoding the enzyme molecule.

### Methods of Making Recombinant Products Using Host (Production) Cells

The host cells of the invention, which can also be referred to as production cells, can be used to express the product encoded by the introduced nucleic acid(s). These production cells are typically stably transfected with the first, second, and/or third exogenous nucleic acids (and optionally further exogenous nucleic acids as described herein where more than one product of interest is to be produced, including multiple subunit products) into a cell to make a production cell. In an alternative embodiment the host cells may be transiently transfected with the first, second, and/or third exogenous nucleic acid into a suitable cell.

Recombinant product can be expressed by culturing the production cells of the invention according to any methods known in the art suitable for producing the product, taking into account the methods described below. In some embodiments, the culture media lacks tyrosine or comprises a level of tyrosine that is less than or equal to 0.01 g/L or less than or equal to 50, 20, or 10 µM (e.g., a level of tyrosine that is insufficient for culturing a similar cell not comprising the one or more exogenous nucleic acid(s) encoding one or more enzyme molecule(s) and/or the subject nucleic acid). Culturing comprises culturing the production cell in the presence of a level of tyrosine that is lower than the level required for survival or growth of a cell (e.g., a similar cell to the production cell) not comprising the one or more exogenous nucleic acid(s), e.g., in the absence of tyrosine. Since it may not be necessary to exert a selective pressure on the producer cell line during recombinant product expression, the culture medium may contain tyrosine at various stages during the growth phase and production phase. However since tyrosine is more difficult to handle in cell culture media due to low solubility, it may be advantageous to omit it completely from the cell culture media.

On the other hand, since in the absence of (or in low levels of) tyrosine, cells will consume more phenylalanine, the various media used, such as the feed solutions, may be supplemented with phenylalanine. For example phenylalanine may be included at a level of at least 0.035 g/L. Accordingly, in some embodiments, phenylalanine is provided (e.g., as part of culturing and/or as a component of the culture media) at a level of at least 0.035 g/L. The cells can therefore be cultured in the presence of a level of phenylalanine that is at least 2, 3, 4, 5, 6, 7, 8, or 9 mM. Since high levels of phenylalanine can be inhibitory to cell growth, typically the level of phenylalanine is less than 10 mM, such as less than 9, 8, 7 or 6 mM.

In the case of the CHO PAH enzyme, in one embodiment it is preferred that the phenylalanine levels in the culture medium are from 2 to 9 mM, such as from 2 or 3 mM to 6 or 7 mM phenylalanine, whereas in the case of the human PAH enzyme in one embodiment a preferred range is from 4 to 9 mM phenylalanine. Our results show that the truncated human PAH enzyme provides excellent cell performance when the cell culture medium is supplemented with phenylalanine.

The cells may be subject to an adaption step so they can adjust to higher levels of phenylalanine. In one embodiment, the cells have already been adapted during the selection stages to growth in phenylalanine-supplemented media. Alternatively this could take place in the production or pre-production stages, for example in an N-1 bioreactor which produces inoculum for the N bioreactor. Again, adaptation may performed other a period of time with increasing concentrations of phenylalanine, or the cells can be seeded into cell culture medium already at the final level of supplementation.

In some embodiments, the level of the phenylalanine is established and/or maintained using an auto-adjusting system that detects and/or monitors the level of phenylalanine in the culture and, responsive to the detected level being less than a threshold value, provides the phenylalanine (e.g., until the detected level is greater than or equal to the threshold value). In some embodiments, such an auto-adjusting system utilizes spectroscopy (e.g., Raman spectroscopy) to detect and/or monitor the level of the phenylalanine. Similar considerations apply where an additional selection marker is used.

In embodiments, the cell culture is carried out as a batch culture, fed-batch culture, abridged fed batch overgrow (aFOG), draw and fill culture, a continuous culture, or semi-continuous culture, including perfusion culture. In some embodiments, a bioreactor is capable of or configured to operate continuously or semi-continuously. In an embodiment, the cell culture is a suspension culture. In one embodiment, the cell or cell culture is placed *in vivo* for expression of the recombinant polypeptide, e.g., placed in a model organism or a human subject. In some embodiments, the cell culture utilizes solid microcarriers (e.g., growth on the surface of a solid microcarrier), porous microcarriers (e.g., growth on and/or within a microcarrier), or support matrices (e.g., growth on and/or within the matrices). In some embodiments, the cell culture is a perfusion culture. In some embodiments, the cell culture is shaken. In some embodiments, the cell culture is a microfluidic culture.

In one embodiment, the culture media is free of serum. Serum-free, protein-free, and chemically-defined animal component-free (CDACF) media are commercially available, e.g., Lonza Bioscience.

In some embodiments, lipid additives (e.g., comprising cholesterol, oleic acid, linoleic acid, or combinations thereof) can be added to the culture media.

Suitable media and culture methods for mammalian cell lines are well-known in the art, e.g., as described in U.S. Pat. No. 5,633,162. Examples of standard cell culture media for laboratory flask or low density cell culture and being adapted to the needs of particular cell types are for instance: Roswell Park Memorial Institute (RPMI) 1640 medium (Morre, G., The Journal of the American Medical Association, 199, p. 519 f. 1967), L-15 medium (Leibovitz, A. et al., Amer. J. of Hygiene, 78, 1p. 173 ff, 1963), Dulbecco's modified Eagle's medium (DMEM), Eagle's minimal essential medium (MEM), Ham's F12 medium (Ham, R. et al., Proc. Natl. Acad. Sc.53, p288 ff. 1965) or Iscoves' modified DMEM lacking albumin, transferrin and lecithin (Iscoves et al., J. Exp. med. 1, p. 923 ff., 1978). For instance, Ham's F10 or F12 media were specially designed for CHO cell culture. Other media specially adapted to CHO cell culture are described in EP481 791. It is known that such culture media can be supplemented with fetal bovine serum (FBS, also called fetal calf serum FCS), the latter providing a natural source of a plethora of hormones and growth factors. The cell culture of mammalian cells is nowadays a routine operation well-described in scientific textbooks and manuals, it is covered in detail e.g. in R. lan Fresney, Culture of Animal cells, a manual, 4th edition, Wiley-Liss/N.Y., 2000. Any of the cell culture media described herein can be formulated to lack a particular amino acid, e.g., the amino acid for which biosynthesis can be rescued if the cell has taken up the subject nucleic acid, such as tyrosine.

Other suitable cultivation methods are known to the skilled artisan and may depend upon the recombinant polypeptide product and the host cell utilized. It is within the skill of an ordinarily skilled artisan to determine or optimize conditions suitable for the expression and production of the recombinant or therapeutic polypeptide to be expressed by the cell.

In one aspect, the disclosure is directed to a method of making or manufacturing a polypeptide product, wherein the method comprises harvesting the polypeptide product. In some embodiments, harvesting comprises separating the polypeptide product from the production cell and/or culture media, e.g., by a method described herein or known in the art.

Culturing may comprises different culture steps. Thus, in some embodiments, the culture steps comprises culturing the production cell in a first culture medium and then in a second culture medium (i.e. using different media which for example may have different levels of tyrosine and/or phenylalanine).

The production cells may be cultured in any suitable vessel at various scales. For industrial production a bioreactor may be used, such as a bioreactor having a volume of at least 10 litres, such as at least 50 litres, 50 to 800 liters, or 800-200,000 liters. A bioreactor may be a single use bioreactor. In embodiments, the bioreactor comprises a bioprocess container, a shell, at least one agitator, at least one sparger, at least one gas filter inlet port for the sparger(s) and headspace overlay, at least one fill port, at least one harvest port, at least one sample port, and at least one probe. A bioreactor may also comprise processes and probes for monitoring and maintaining one or more parameters, e.g., pH, dissolved oxygen tension (DOT), phenylalanine levels and/or temperature. The bioreactor may be operably coupled to a harvest vessel. Further details and embodiments are provided in the 'Applications' section below.

Once biosynthesis of the product by the production cells has progressed to a satisfactory point, the product can be harvested e.g. withdrawing culture medium and separating the supernatant from cells and cell debris. The product can be subject to one or more purification/treatment steps to obtain purified product, such as affinity chromatography, ion exchange chromatography, filtration and/or viral inactivation. The product may also be combined with one or more pharmaceutically acceptable carriers, excipients or diluents to produce a composition such as a formulated pharmaceutical composition e.g. with one or more of a buffer, a surfactant, a stabilizer (such as trehalose, sucrose, glycerol), an amino acid (such as glycine, histidine, arginine), metal ions/chelators, salts and/or a preservative.

### Recombinant Products

The products encompassed by the present disclosure include, but are not limited to, molecules, nucleic acids (e.g., non-coding nucleic acids, e.g., non-coding RNA molecules, e.g., an antisense RNA, siRNA, tRNA, ribosomal RNA, microRNA, piRNA, snoRNA, snRNA, exRNA, scaRNA, or long noncoding RNA, e.g., Xist or HOTAIR), polypeptides (e.g., recombinant and/or therapeutic polypeptides), or hybrids thereof, that can be produced by, e.g., expressed in, a cell. In some embodiments, the cells are engineered or modified to produce the product. Such modifications include introducing molecules that control or result in production of the product. For example, a cell is modified by introducing an exogenous nucleic acid that encodes a polypeptide, e.g., a recombinant polypeptide, and the cell is cultured under conditions suitable for production, e.g., expression and secretion, of the polypeptide, e.g., recombinant polypeptide. In another example, a cell is modified by introducing an exogenous nucleic acid that controls, e.g., increases, expression of a polypeptide that is endogenously expressed by the cell, such that the cell produces a higher level or quantity of the polypeptide than the level or quantity that is endogenously produced, e.g., in an unmodified cell. In embodiments, the cell or cell line identified, selected, or generated by the methods described herein produces a product, e.g., a recombinant polypeptide, useful in the treatment of a medical condition, disorder or disease.

### Polypeptides

In some embodiments, the product of interest comprises one or more polypeptides, e.g., a recombinant polypeptide, which is typically is a heterologous polypeptide i.e. a product that is not naturally expressed by the cell. The product can be a therapeutic protein or a diagnostic protein, e.g., useful for drug screening. The therapeutic or diagnostic protein can be an antibody molecule, e.g., an antibody or an antibody fragment, a fusion protein, a hormone, a cytokine, a growth factor, an enzyme, a glycoprotein, a lipoprotein, a reporter protein, a therapeutic peptide, an aptamer, or a structural and/or functional fragment or hybrid of any of these. In one embodiment, the product comprises multiple polypeptide chains, e.g., an antibody or antibody fragment that comprises a heavy and a light chain.

In some embodiments, the product is an antibody molecule. Products encompassed herein are diagnostic antibody molecules, e.g., a monoclonal antibody or antibody fragment thereof, useful for imaging techniques, and therapeutic antibody molecules suitable for administration to subjects, e.g., useful for treatment of diseases or disorders. An antibody molecule is a protein, or polypeptide sequence derived from an immunoglobulin molecule which specifically binds with an antigen. In an embodiment, the antibody molecule is a full-length antibody or an antibody fragment. Antibodies and multiformat proteins can be polyclonal or monoclonal, multiple or single chain, or intact immunoglobulins, and may be derived from natural sources or from recombinant sources. Antibodies can be multimers of immunoglobulin molecules, e.g., tetramers of immunoglobulin molecules. In an embodiment, the antibody is a monoclonal antibody. The antibody may be a human or humanized antibody. In one embodiment, the antibody is an IgA, IgG, IgD, IgM, or IgE antibody. In one embodiment, the antibody is an IgG1, IgG2, IgG3, or IgG4 antibody. In some embodiments, the antibody molecule is or comprises a multi-specific antibody, e.g., a bi-, tri-, or tetra-specific antibody, e.g., a BiTE.

"Antibody fragment" refers to at least one portion of an intact antibody, or recombinant variants thereof, and refers to the antigen binding domain, e.g., an antigenic determining variable region of an intact antibody, that is sufficient to confer recognition and specific binding of the antibody fragment to a target, such as an antigen. Examples of antibody fragments include, but are not limited to, Fab, Fab', F(ab')₂, and Fv fragments, scFv antibody fragments, linear antibodies, single domain antibodies such as sdAb (either VL or VH), camelid VHH domains, and multi-specific antibodies formed from antibody fragments such as a bivalent fragment comprising two Fab fragments linked by a disulfide bridge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

Examples of polypeptides of interest include, but are not limited to, those listed below:
Hormones: Erythropoietin, Epoein-α, Darbepoetin-α, Growth hormone (GH), somatotropin, Human follicle-stimulating hormone (FSH), Human chorionic gonadotropin, Lutropin-α, Glucagon, Growth hormone releasing hormone (GHRH), insulin.
Blood Clotting/Coagulation Factors: Factor VIIa, Factor VIII, Factor IX, Antithrombin III (AT-III), Protein C concentrate
Cytokine/Growth Factors: Type I alpha-interferon, Interferon-αn3 (IFNαn3), Interferon-β1a (rIFN- β), Interferon-β1b (rIFN- β), Interferon-y1b (IFN y), Aldesleukin (interleukin 2(IL2), epidermal theymocyte activating factor; ETAF, Palifermin (keratinocyte growth factor; KGF), Becaplemin (platelet-derived growth factor; PDGF), Anakinra (recombinant IL1 antagonist).
Antibodies: Bevacizumab (VEGFA mAb), Cetuximab (EGFR mAb), Panitumumab (EGFR MAb), Alemtuzumab (CD52 mAb), Rituximab (CD20 chimeric Ab), Trastuzumab, Adalimumab, infliximab, Tositumomab, Acritumomab, Ranibizumab, Abciximab, Omalizumab, Palivizumab, Natalizumab, Daclizumab, Basiliximab, Eculizumab.
Vaccine antigens: Hepatitis B surface antigen (HBsAg), HPV antigens, HIV antigens, influenza antigens.
Others: Albumin, Anti-Rhesus (Rh) immunoglobulin G, Enfuvirtide, Spider silk proteins e.g., fibrion, botulinum toxin type A, alglucerase, imiglucerase, recombinant human hyaluronidase, Palifermin, Anakinra, dornase alfa, synthetic porcine secretin.

The recombinant polypeptide of interest may be a multispecific protein, e.g., a bispecific antibody, of which numerous formats are available such as BsIgG (Triomab), BiTE, DART, TandB.

In some embodiments, the polypeptide (e.g., produced by a cell and/or according to the methods described herein) is an antigen expressed by a cancer cell. In some embodiments the recombinant or therapeutic polypeptide is a tumor-associated antigen or a tumor-specific antigen. In some embodiments, the recombinant or therapeutic polypeptide is selected from HER2, CD20, 9-O-acetyl-GD3, βhCG, A33 antigen, CA19-9 marker, CA-125 marker, calreticulin, carboanhydrase IX (MN/CA IX), CCR5, CCR8, CD19, CD22, CD25, CD27, CD30, CD33, CD38, CD44v6, CD63, CD70, CC123, CD138, carcinoma embryonic antigen (CEA; CD66e), desmoglein 4, E-cadherin neoepitope, endosialin, ephrin A2 (EphA2), epidermal growth factor receptor (EGFR), epithelial cell adhesion molecule (EpCAM), ErbB2, fetal acetylcholine receptor, fibroblast activation antigen (FAP), fucosyl GM1, GD2, GD3, GM2, ganglioside GD3, Globo H, glycoprotein 100, HER2/neu, HER3, HER4, insulin-like growth factor receptor 1, Lewis-Y, LG, Ly-6, melanoma-specific chondroitin-sulfate proteoglycan (MCSCP), mesothelin, MUCI, MUC2, MUC3, MUC4, MUC5_{AC}, MUC5_{B}, MUC7, MUC16, Mullerian inhibitory substance (MIS) receptor type II, plasma cell antigen, poly SA, PSCA, PSMA, sonic hedgehog (SHH), SAS, STEAP, sTn antigen, TNF-alpha precursor, and combinations thereof.

In some embodiments, the polypeptide (e.g., produced by a cell and/or according to the methods described herein) is an activating receptor and is selected from 2B4 (CD244), α₄β₁ integrin, β₂ integrins, CD2, CD16, CD27, CD38, CD96, CDIOO, CD160, CD137, CEACAMI (CD66), CRTAM, CSI (CD319), DNAM-1 (CD226), GITR (TNFRSF18), activating forms of KIR, NKG2C, NKG2D, NKG2E, one or more natural cytotoxicity receptors, NTB-A, PEN-5, and combinations thereof, optionally wherein the β₂ integrins comprise CD11a-CD 18, CD11 b-CD 18, or CD11c-CD 18, optionally wherein the activating forms of KIR comprise KIR2DSI, KIR2DS4, or KIR-S, and optionally wherein the natural cytotoxicity receptors comprise NKp30, NKp44, NKp46, or NKp80.

In some embodiments, the polypeptide (e.g., produced by a cell and/or according to the methods described herein) is an inhibitory receptor and is selected from KIR, ILT2/LIR-I/CD85j, inhibitory forms of KIR, KLRG1, LAIR-1, NKG2A, NKR-P1A, Siglec-3, Siglec-7, Siglec-9, and combinations thereof, optionally wherein the inhibitory forms of KIR comprise KIR2DL1, KIR2DL2, KIR2DL3, KIR3DL1, KIR3DL2, or KIR-L.

In some embodiments, the polypeptide (e.g., produced by a cell and/or according to the methods described herein) is an activating receptor and is selected from CD3, CD2 (LFA2, OX34), CD5, CD27 (TNFRSF7), CD28, CD30 (TNFRSF8), CD40L, CD84 (SLAMF5), CD137 (4-1BB), CD226, CD229 (Ly9, SLAMF3), CD244 (2B4, SLAMF4), CD319 (CRACC, BLAME), CD352 (Lyl08, NTBA, SLAMF6), CRTAM (CD355), DR3 (TNFRSF25), GITR (CD357), HVEM (CD270), ICOS, LIGHT, LTβR (TNFRSF3), OX40 (CD134), NKG2D, SLAM (CD150, SLAMF1), TCRα, TCRβ, TCRδy, TIM1 (HAVCR, KIM1), and combinations thereof.

In some embodiments, the polypeptide (e.g., produced by a cell and/or according to the methods described herein) is an inhibitory receptor and is selected from PD-1 (CD279), 2B4 (CD244, SLAMF4), B71 (CD80), B7HI (CD274, PD-L1), BTLA (CD272), CD160 (BY55, NK28), CD352 (Ly108, NTBA, SLAMF6), CD358 (DR6), CTLA-4 (CD152), LAG3, LAIR1, PD-1H (VISTA), TIGIT (VSIG9, VSTM3), TIM2 (TIMD2), TIM3 (HAVCR2, KIM3), and combinations thereof.

Other recombinant protein products (e.g., produced by a cell and/or according to the methods described herein) include non-antibody scaffolds or alternative protein scaffolds, such as, but not limited to: DARPins, affibodies and adnectins. Such non-antibody scaffolds or alternative protein scaffolds can be engineered to recognize or bind to one or two, or more, e.g., 1, 2, 3, 4, or 5 or more, different targets or antigens.

### Applications

The present disclosure features, inter alia, production cells, methods of making or manufacturing a polypeptide product using production cells, methods of identifying, selecting, and/or culturing a cell (e.g., a production cell), and method of making or producing a production cell. The methods of identifying, selecting, and/or culturing cells as disclosed herein can be used to generate cells, e.g., production cells, useful for producing a variety of products, evaluate various cell lines, or to evaluate the production of various cell lines for use in a bioreactor or processing vessel or tank, or, more generally with any feed source. The compositions and methods described herein are suitable for culturing eukaryotic cell lines. Further, in embodiments, the compositions and methods described herein are suitable for culturing suspension cells or anchorage-dependent (adherent) cells and are suitable for production operations configured for production of pharmaceutical and biopharmaceutical products-such as polypeptide products, nucleic acid products (for example DNA or RNA), exosomes, vesicles, or cells and/or viruses such as those used in cellular and/or viral therapies or as vaccines.

In embodiments, the cells, e.g., production cells, express or produce a product, such as a recombinant therapeutic or diagnostic product. As described in more detail below, examples of products produced by cells include, but are not limited to, antibody molecules (e.g., monoclonal antibodies, bispecific antibodies), antibody mimetics (polypeptide molecules that bind specifically to antigens but that are not structurally related to antibodies such as e.g. DARPins, affibodies, adnectins, or IgNARs), fusion proteins (e.g., Fc fusion proteins, chimeric cytokines), other recombinant proteins (e.g., glycosylated proteins, enzymes, hormones), viral therapeutics (e.g., anti-cancer oncolytic viruses, viral vectors for gene therapy and viral immunotherapy), cell therapeutics (e.g., pluripotent stem cells, mesenchymal stem cells and adult stem cells), vaccines or lipid-encapsulated particles (e.g., exosomes, virus-like particles), RNA (such as e.g. siRNA) or DNA (such as e.g. plasmid DNA), antibiotics or amino acids. In embodiments, the compositions and methods described herein can be used for producing biosimilars.

As mentioned, in embodiments, compositions and methods described herein allow for the production of eukaryotic cells, e.g., mammalian cells or lower eukaryotic cells such as for example yeast cells or filamentous fungi cells and/or products of the eukaryotic cells, e.g., proteins, peptides, antibiotics, amino acids, nucleic acids (such as DNA or RNA), synthesized by the eukaryotic cells in a large-scale manner. Unless stated otherwise herein, the compositions and methods described herein can include any desired volume or production capacity including but not limited to bench-scale, pilot-scale, and full production scale capacities.

Moreover and unless stated otherwise herein, the compositions and methods described herein can be used with any suitable reactor(s) including but not limited to stirred tank, airlift, fiber, microfiber, hollow fiber, ceramic matrix, fluidized bed, fixed bed, and/or spouted bed bioreactors with or without use of solid or porous microcarriers or supports. As used herein, "reactor" can include a fermenter or fermentation unit, or any other reaction vessel and the term "reactor" is used interchangeably with "fermenter." For example, in some aspects, a bioreactor unit can perform one or more, or all, of the following: feeding of nutrients and/or carbon sources, injection of suitable gas (e.g., oxygen), inlet and outlet flow of fermentation or cell culture medium, separation of gas and liquid phases, maintenance of temperature, maintenance of oxygen and CO₂ levels, maintenance of pH level, agitation (e.g., stirring), and/or cleaning/sterilizing. Example reactor units, such as a fermentation unit, may contain multiple reactors within the unit, for example the unit can have 1, 2, 3, 4, 5, 10, 15, 20, 25, 30, 35, 40, 45, 50, 60, 70, 80, 90, or 100, or more bioreactors in each unit and/or a facility may contain multiple units having a single or multiple reactors within the facility. In various embodiments, the bioreactor can be suitable for batch, semi fed-batch, fed-batch, perfusion, and/or a continuous fermentation processes. Any suitable reactor diameter can be used. In embodiments, the bioreactor can have a volume between about 100 ml and about 50,000 L. Non-limiting examples include a volume of 10 ml, 50 ml, 100 ml, 250 ml, 500 ml, 750 ml, 1 liter, 2 liters, 10 liters, 50 liters, 100 liters, 500 liters, 1000 liters, 2000 liters, 5000 liters, 10,000 liters, 15,000 liters, 20,000 liters, and/or 50,000 liters, or approximately those volumes. In the context of industrial scale manufacture required to make sufficient product for clinical or commercial use, the volume is typically at least 10 litres. In some embodiments, the bioreactor is configured to grow a microfluidic culture. Additionally, suitable reactors can be multi-use, single-use, disposable, or non-disposable and can be formed of any suitable material including metal alloys such as stainless steel (e.g., 316 L or any other suitable stainless steel) and Inconel, plastics, and/or glass. In some embodiments, suitable reactors can be round, e.g., cylindrical. In some embodiments, suitable reactors can be square, e.g., rectangular. Square reactors may in some cases provide benefits over round reactors such as ease of use (e.g., loading and setup by skilled persons), greater mixing and homogeneity of reactor contents, and lower floor footprint.

In embodiments and unless stated otherwise herein, the compositions and methods described herein can be used with any suitable unit operation and/or equipment not otherwise mentioned, such as operations and/or equipment for separation, purification, and isolation of such products. Any suitable facility and environment can be used, such as traditional stick-built facilities, modular, mobile and temporary facilities, or any other suitable construction, facility, and/or layout. For example, in some embodiments modular clean-rooms can be used. Additionally and unless otherwise stated, the compositions and methods described herein can be housed and/or performed in a single location or facility or alternatively be housed and/or performed at separate or multiple locations and/or facilities.

By way of non-limiting examples and without limitation, U.S. Publication Nos. 2013/0280797; 2012/0077429; 2011/0280797; 2009/0305626; and U.S. Patent Nos. 8,298,054; 7,629,167; and 5,656,491, describe exemplary facilities, equipment, and/or systems that may be suitable for use with the compositions and methods described herein.

In a preferred embodiment the mammalian cells are CHO-cell lines. Examples include a CHO-K1 cell, a CHO-K1 SV cell, a DG44 CHO cell, a DUXB11 CHO cell, a CHOS, a CHO GS knock-out cell, a CHO FUT8 GS knock-out cell, a CHOZN, and a CHO-derived cell. The CHO GS knock-out cell (e.g., GSKO cell) is, for example, a CHOK1SV^{®} GS knockout cell. The CHO FUT8 knockout cell is, for example, the Potelligent^{®} CHOK1SV^{®} (Lonza Biologics, Inc.).

In one embodiment, the eukaryotic cell is a lower eukaryotic cell such as e.g. a yeast cell (e.g., *Pichia* genus (e.g. *Pichia pastoris, Pichia methanolica, Pichia kluyveri,* and *Pichia angusta*)*, Komagataella* genus (e.g. *Komagataella pastoris, Komagataella pseudopastoris* or *Komagataella phaffii*)*, Saccharomyces* genus (e.g. *Saccharomyces cerevisae, Saccharomyces kluyveri, Saccharomyces uvarum), Kluyveromyces* genus (e.g. *Kluyveromyces lactis, Kluyveromyces marxianus*)*,* the *Candida* genus (e.g. *Candida utilis, Candida cacaoi, Candida boidinii*), the *Geotrichum* genus (e.g. *Geotrichum fermentans*)*, Hansenula polymorpha, Yarrowia lipolytica,* or *Schizosaccharomyces pombe.* Preferred is the species *Pichia pastoris.* Examples for *Pichia pastoris* strains are X33, GS115, KM71, KM71H; and CBS7435.

In embodiments, the cultured cells are used to produce proteins e.g., antibodies, e.g., monoclonal antibodies, and/or recombinant proteins, for therapeutic use. In embodiments, the cultured cells produce peptides, amino acids, fatty acids or other useful biochemical intermediates or metabolites. For example, in embodiments, molecules having a molecular weight of about 4000 daltons to greater than about 140,000 daltons can be produced. In embodiments, these molecules can have a range of complexity and can include posttranslational modifications including glycosylation.
The present invention will be illustrated further with reference to the following examples, which are non-limiting.

### EXAMPLES

### Example 1: Materials and Methods

### Cell Culture

Suspension Lonza CHOK1SV^{®} GS-KO^{®} cells were maintained in CD-CHO medium (Gibco 10743-029) supplemented with 6 mM L-glutamine (Sigma G8540). These were incubated at 37°C at 140 rpm in a 5% CO₂ atmosphere. Cells were seeded at 0.2 x 10⁶ viable cells/ml in 125 ml Erlenmeyer flasks in 20 ml, these were passaged every 3-4 days.

### Reversion assay

Lonza CHOK1SV^{®} GS-KO^{®} cells were seeded at 5000 viable cells per well in 200 µl medium in 96 well plates and analyzed for outgrowth after 11 days and 3 weeks. CD CHO with no tyrosine and Lonza CM76 (Lonza Biologics plc) with no tyrosine but supplemented with 6 mM L-glutamine were used as test media. 7.2x10⁶ viable cells were tested in CM76 no tyrosine medium and 2.4 x10⁶ cells were tested in CD CHO no tyrosine medium. Complete medium (CD-CHO + L-glut) was used as a positive control whilst medium void of L-glutamine (CD-CHO only) was used as a negative control.

### Plasmids and transfection to make stable cell lines

**Table 1 - Vector Constructs**

| | **Expression Cassette 1** | | **Expression Cassette 2** | | **Expression Cassette 3** | |
|---|---|---|---|---|---|---|
| Vector Name | **Promoter** | **Selection Gene** | **Promoter** | **Recombinant Gene 1** | **Promoter** | **Selection Gene** |
| LMM170 | SV40 | Glutamine Synthetase (Lonza) | mCMV | eGFP | SV40 | GCH1 |
| LMM172 | SV40 | truncated PAH CHO | mCMV | eGFP | SV40 | GCH1 |
| LMM173 | SV40 | truncated PAH human | mCMV | eGFP | SV40 | GCH1 |
| LMM182 | SV40 | truncated PAH CHO | | | PGK | GCH1 |
| LMM183 | SV40 | truncated PAH CHO | | | SV40 | GCH1 |
| LMM184 | SV40 | truncated PAH CHO | | | mCMV | GCH1 |
| LMM185 | SV40 | truncated PAH human | | | PGK | GCH1 |
| LMM186 | SV40 | truncated PAH human | | | SV40 | GCH1 |
| LMM187 | SV40 | truncated PAH human | | | mCMV | GCH1 |

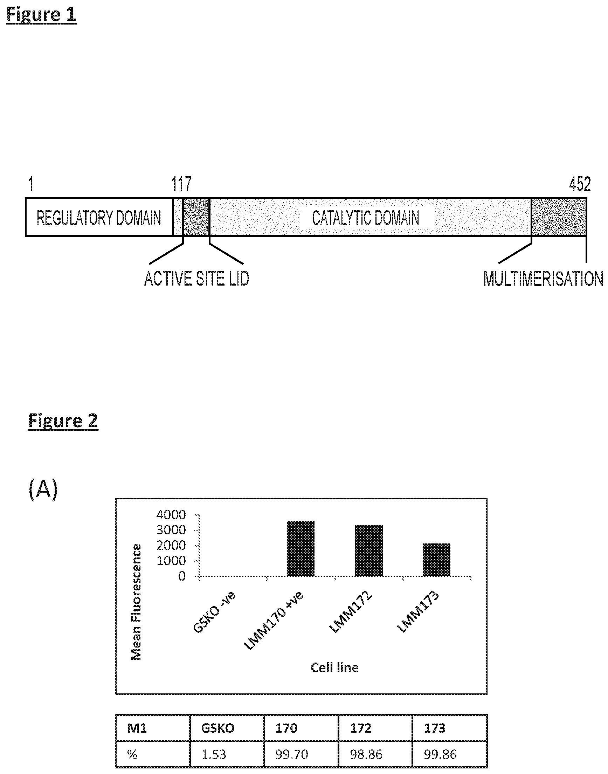

The truncated PAH sequences have a deletion of the N-terminal 116 amino acids that contain a regulatory domain (Daubner SC *et al.,* 1997, ibid). The various domains of PAH are shown in Figure 1.

Plasmids were linearized with Pvul (NEB, R3150L) and purified using an ethanol precipitation protocol. Electroporation was carried out on a Biorad Genepulser Xcell electroporator. 20 µg of linearized plasmid in 100 µl TE buffer and 1x10⁷ viable Lonza CHOK1SV GS-KO cells/ 700 µl CM76 no tyrosine (+6 mM L-glut) medium was added to an electroporation cuvette. The DNA cell mix was electroporated at 300 V and 900 µF with a cuvette diameter of 0.4 mm. 1 ml of prewarmed medium was added to the cuvette immediately after electroporation. The cells were then transferred to 2 x 5 ml CM76 no tyrosine (+6 mM L-glut) medium in T25 flasks. The flasks were incubated at 37°C in a static incubator with a 5% CO₂ gas environment. Post 24 hours, an additional 5 ml of CM76 tyrosine free (+6 mM L-glut) medium was added to the T25 flasks. Cell counts were carried out using a ViCell instrument 21 days post transfection to assess transfection success. Further confirmation that transfection was successful was undertaken by visualizing cells growing in T25 flasks under a microscope (Leica MZFLIII with GFP2 filter, x100 magnification) for eGFP expression.

### Growth curve profiles and culture viability

Cells were seeded at 0.2 x 10⁶ cell/ml in 20 ml in a 125 ml Erlenmeyer flask and shaken at 140 rpm, 37°C, in a 5% CO₂ environment. Readings were recorded every 48 hours for the number of days indicated in the example figures using a ViCell (Beckman Coulter) instrument where 0.2 ml of sample with 0.8 ml pre-warmed PBS was used to determine viable cell concentrations and cell diameter.

### FACS

1 x 10⁵ cells were pelleted in a centrifuge at 1,000 rpm for 5 minutes and resuspended in 350 µl PBS. Samples were then loaded onto the probe of a FACScalibur^{™} (BD biosciences) and fluorescence intensity was measured in relation to the cell count. The forward scatter (FSC) was measured using the E-1 amplifier and side scatter (SSC) set to 465 whilst FL1 recorded cells at 473; all settings were converted to Log scales.

### SDS-PAGE, western blot

1x10⁶ cells were pelleted in a centrifuge at 1000 rpm 5min and lysed in 100 µl of ice-cold lysis buffer consisting of 20 mM HEPES-NaOH, pH 7.2, 100 mM NaCl, 10 mM Na β-glycerophosphate, 0.5% Nonidet-P40 with 50 mM NaF, 1 mM activated Na₃ VO₄, 10 µg/ml leupeptin, 2 µg/ml pepstatin and 0.2 mM PMSF added just before use.

10 µg of reduced protein sample or 10 µl of non-reduced supernatant sample was run on 10% SDS-PAGE acrylamide gels and western transfers on to nitrocellulose were undertaken as previously described (Roobol, Carden et al., 2009, FEBS J. 276: 286-302). Antibodies were sourced from Sigma (anti-GCH1, SAB1405858-50µg, anti-PAH, HPA031642, anti-GS G2781, anti-B-actin A5441, anti-Human IgG (γ-chain specific) I9764) and from CRUK (eGFP 3E1). Anti-tubulin (Woods, Sherwin et al., 1989, J. Cell Sci. 93: 491-500) was a kind gift from Professor Keith Gull, University of Oxford, UK while anti-L7a was generated against the N-terminal sequence of human L7a (Roobol and Carden, 1999, Eur. J. Cell Biol. 78(1): 21-32). Secondary antibodies for immunoblot detection of cell lysate proteins were anti-whole IgG (mouse or rabbit)-HRP conjugates (Sigma) followed by ECL (GE Healthcare) detection.

### qRTPCR

1x10⁸ viable cells were harvested for RNA extraction and mRNA amounts determined by qRTPCR using the Qiagen Quantifast kit with the following primer sets; PAH (qrtPAHtotfwd CATCAAGGCATATGGTGCTG (SEQ ID NO: 7) & qrtPAHtotrvs GGGCTGGAACTCTGTGACAT ((SEQ ID NO: 8)), GCH1 (GCH1fwd: CTTCACCAAGGGCTACCAGG ((SEQ ID NO: 9); GCH1 rev: AGGCCAAGGACTTGCTTGTT (SEQ ID NO: 10)) and β-actin (CHObactqF AGCTGAGAGGGAAATTGTGCG (SEQ ID NO: 11) & CHObactqR GCAACGGAACCGCTC ATT (SEQ ID NO: 12)) on a Eppendorf RealPlex cycler instrument.

### Example 2: Reversion assay of GSKO cells grown in either CM76 or CD CHO media with no tyrosine but supplemented with 6mM L-glutamine

This example demonstrates the low reversion rate observed when growing exemplary cells unable to grow in the absence of tyrosine.

CHOK1SV GS-KO^{®} host cells were seeded into 96 well plates in medium lacking tyrosine but supplemented with 6 mM glutamine. A positive control was CHOK1SV GS-KO^{®} host cells growing in medium supplemented with 6 mM glutamine and the negative control was medium lacking glutamine. The results are shown below in Table 2. In medium lacking tyrosine, no reversion colonies/cell growth was observed and the plate looked similar to the negative control. This suggests that a tyrosine auxotrophy marker would be a useful selective marker in a production cell.

**Table 2**

| | **CM76 no tyr + 6mM Glutamine** | | **CD CHO no tyr + 6mM Glutamine** | |
|---|---|---|---|---|
| | Reversion Rate: 11 days | Reversion Rate: 3 weeks | Reversion Rate: 11 days | Reversion Rate: 3 weeks |
| **+ve Control** | 100% wells grew | 100% wells grew | 100% wells grew | 100% wells grew |
| **-ve Control** | 0% wells grew | 0% wells grew | 0% wells grew | 0% wells grew |
| **No Tyrosine 5,000 cells/well** | 0/ 7.2 X10⁶ cells | 0/ 7.2 X10⁶ cells | 0/ 2.4 X10⁶ cells | 0/ 2.4 X10⁶ cells |

### Example 3: Growth of Exemplary Production Cells in the Absence of Tyrosine

This example demonstrates that cells lacking exogenous nucleic acids encoding PAH and GCH1 enzyme molecules do not grow in the absence of tyrosine, whereas exemplary production cells containing vectors LMM172 or LMM173 comprising exogenous nucleic acids encoding both PAH and GCH1 enzyme molecules are observed to grow in the absence of tyrosine and express a report molecule eGFP. However, when we initially tested full length CHO PAH together with GCH1, we found that recovery of transfected cells in tyrosine-free media was either very slow (CD CHO), or where CM76 medium was used, there was no recovery at all (data not shown). We therefore tried a truncated version of PAH where the N-terminal 116 amino acids which encode a regulatory domain have been removed.

The vectors used to generate these pools contained a truncated version of PAH (cassette 1, tPAH with first 116 amino acids deleted with the sequence derived from either CHO cells or human and driven by an SV40 promoter), GCH1 (cassette 3 driven by an SV40 promoter) and eGFP (cassette 2, driven by a CMV promoter). Two controls were included where the 1^{st} cassette contained a glutamine synthetase (GS) gene driven by an SV40 promoter (vector LMM170). Transfected controls were either grown in the absence of tyrosine (negative control) or the presence of tyrosine (positive control).

CHOK1SV GS-KO^{®} host cells were transfected via electroporation with the linearized vectors and subsequently cultured for three weeks in media without tyrosine but supplemented with 6 mM glutamine (except the positive control which also included tyrosine).

CHOK1SV GS-KO^{®} host engineered cells were shown to grow successfully in tyrosine free medium only when the truncated PAH and GCH1 were co-expressed. In addition, when these components were transfected individually, cells did not survive transfection and grow in Tyr-free medium (data not shown). Thus, both the PAH enzyme molecule comprising truncated PAH and GCH1 enzyme molecule are required to support exemplary CHO production cell growth in the absence of tyrosine.

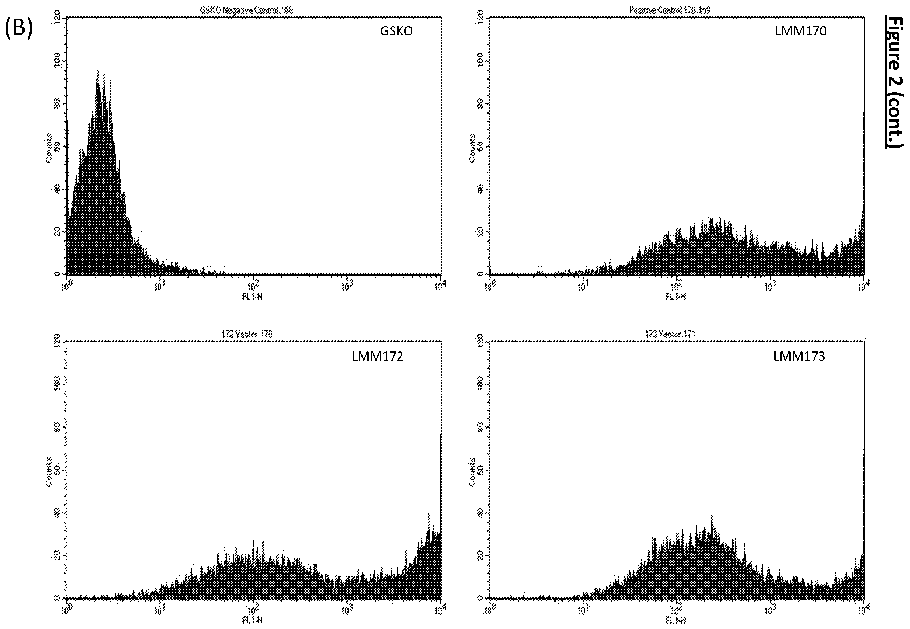

Fig. 2 shows histograms obtained using flow cytometry of the mean fluorescence from the population of cells after transfection and recovery for 3 weeks and confirms eGFP expression in cells growing in tyrosine free medium. The mean fluorescence from exemplary production cells comprising the truncated CHO cell derived PAH sequence and GCH1 (vector LMM172) was similar to that from the GS positive control (vector LMM170 +ve). The production cells containing the CHO truncated PAH sequence (LMM172) showed a higher GFP expression compared to the human truncated PAH sequence (LMM173). This is a model where recombinant proteins could replace eGFP if the PAH and GCH1 combined system was used as a selection marker.

### Example 4: PAH Protein and mRNA Amounts

This example demonstrates that exemplary production cells containing vector LMM173 comprising exogenous nucleic acids encoding human PAH and GCH1 enzyme molecules exhibit PAH protein and mRNA expression and eGFP protein expression; the example further demonstrates that exemplary production cells containing vector LMM172 comprising exogenous nucleic acids encoding CHO PAH and GCH1 enzyme molecules exhibit PAH mRNA expression and eGFP protein expression.

Western blot analysis of lysates from cells pools from Fig. 2 was performed. The control was grown in media containing 6 mM glutamine and tyrosine, LMM170 cells were grown in media containing tyrosine but not glutamine. LMM172 and LMM173 were grown in tyrosine free medium supplemented with 6 mM glutamine. Tubulin and L7a were used as loading controls. The PAH antibody only detected the human truncated PAH (bands at approximately 37 and 50 kDa) and not the CHO truncated PAH (LMM172) (data not shown). eGFP was confirmed as being expressed in cell pools where the transfected vector contained the eGFP gene in cassette 2.

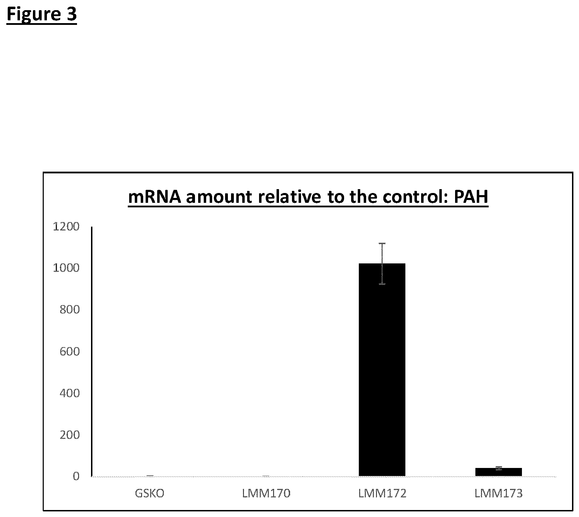

Fig. 3 shows qRT-PCR data which detected expression of truncated CHO PAH and truncated human PAH mRNA expression. The truncated CHO PAH mRNA was expressed to a much greater amount than the truncated human PAH. Both were increased over the controls confirming exogenous PAH mRNA expression in the exemplary production cells.

### Example 5: Growth Profiles and Culture Viability in the Absence of Tyrosine

This example demonstrates that exemplary production cells containing vector LMM172 comprising exogenous nucleic acids encoding CHO PAH and GCH1 enzyme molecules are able to grow to higher viable cell concentrations and have prolonged culture viability than similar cells not comprising the exogenous nucleic acids in the absence of tyrosine.

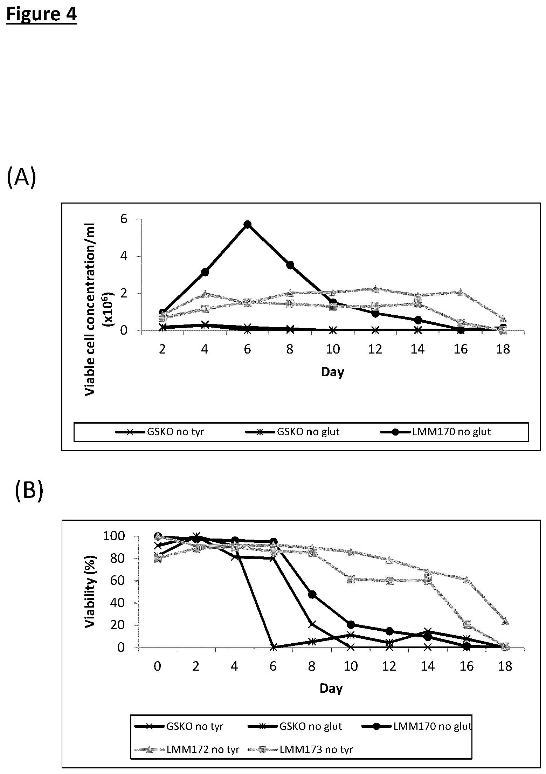

Fig. 4 shows growth data of exemplary production cell pools generated as described in Examples 3 and 4. Cell pools were cultured in 125 ml Erlenmeyer flasks for 18 days in the absence of tyrosine or glutamine. Every two days, the cells were sampled and the number of viable cells and culture viability assessed using a ViCell instrument; no further feeds were introduced. Fig. 4 shows (A) viable cell concentration and (B) culture viability. The CHO cell truncated PAH cell pool (LMM 172) grew to higher cell numbers and had longer culture viability than the human truncated PAH cell pool (LMM 173)

### Example 6: Growth Profiles and Viable Cell Concentrations of Exemplary Production Cells When Grown in Absence of Tyrosine but Supplemented with additional Phenylalanine

This example demonstrates the growth and culture viability characteristics of exemplary production cells comprising exogenous nucleic acids encoding PAH and GCH1 enzyme molecules.

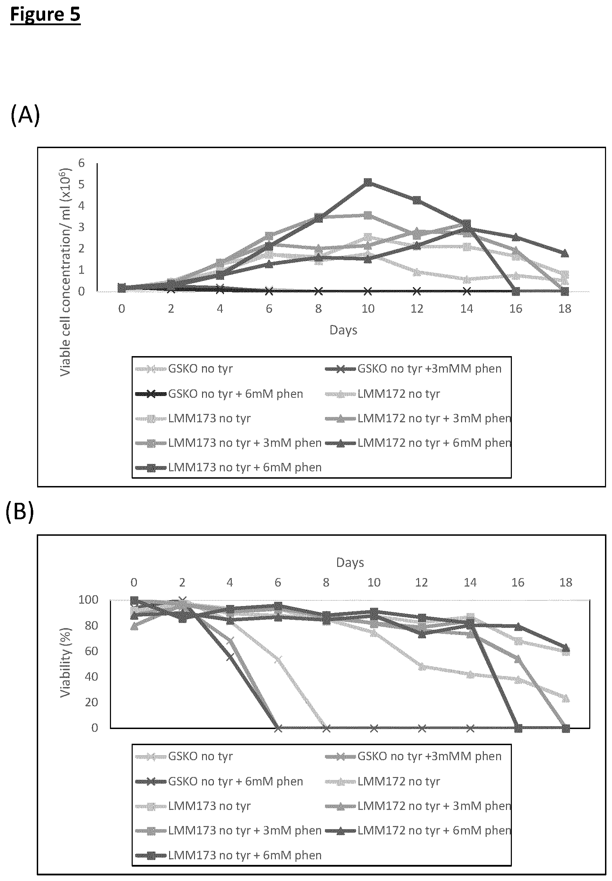

Fig. 5 shows growth data of exemplary production cell pools Cultures were grown in 125 ml Erlenmeyer flasks for 18 days and where indicated were supplemented with phenylalanine (Sigma P5482). Cells were sampled every two days and no further feeds were introduced. These cells were analyzed for cell growth and culture viability. Exemplary production cells expressing truncated human PAH grew to higher viable cell concentrations and reached these in a shorter time than the exemplary production cells expressing truncated CHO PAH when supplemented with 6 mM phenylalanine. This experiment also demonstrated that the cell lines are truly prototrophic as the GSKO controls died

### Example 7: Growth Profiles and Viable Cell Concentrations of Exemplary Production Cells When Grown in Commercial CD-CHO medium Absent of Tyrosine but Supplemented with additional Phenylalanine

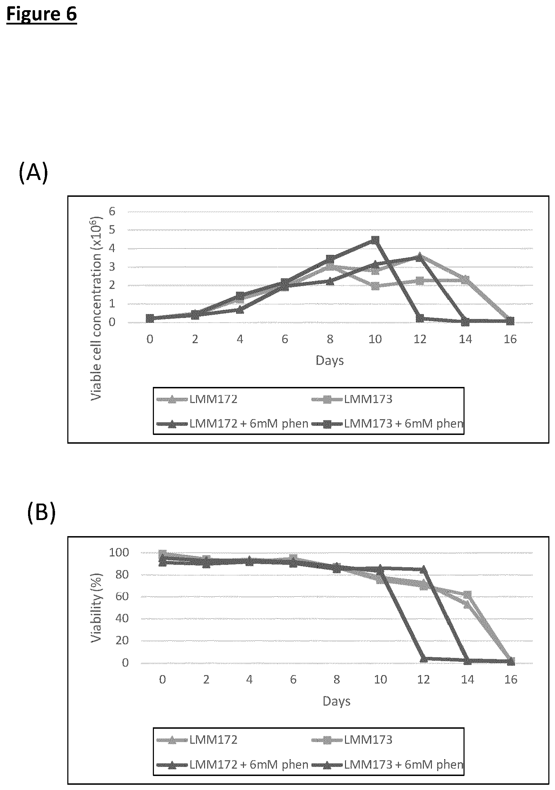

This example evaluates cell growth and culture viability. Fig. 6 shows growth data of exemplary production cells transfected and grown in commercial CD-CHO (ThermoFisher Scientific) media lacking tyrosine but supplemented with 6 mM glutamine. Transfected CHOK1SV GS-KO^{™} host cells recovered faster post transfection in CD CHO medium compared to CM76 medium. The recovery rate was reduced from 21 days to 18 days where cells were ready to transfer to shake flask post transfection. Additionally, cells transfected with plasmid DNA constructs containing the truncated human PAH recovered in a similar time, and to a similar viable cell number, as that observed when using vectors containing the truncated CHO PAH cells post transfection. This indicated that CD CHO is a better transfection medium for this system.

Cells assessed for growth in CD CHO medium were sampled every two days and no further feeds were introduced. The cultures were analyzed for cell growth and culture viability. When tyrosine prototrophic cell pools were grown in CD CHO medium (Fig. 6) the truncated human PAH cell pool benefited most from the additional 6 mM phenylalanine.

### Example 8: Preadapting cells to Phenylalanine Supplementation Reduces the Growth Lag Phase.

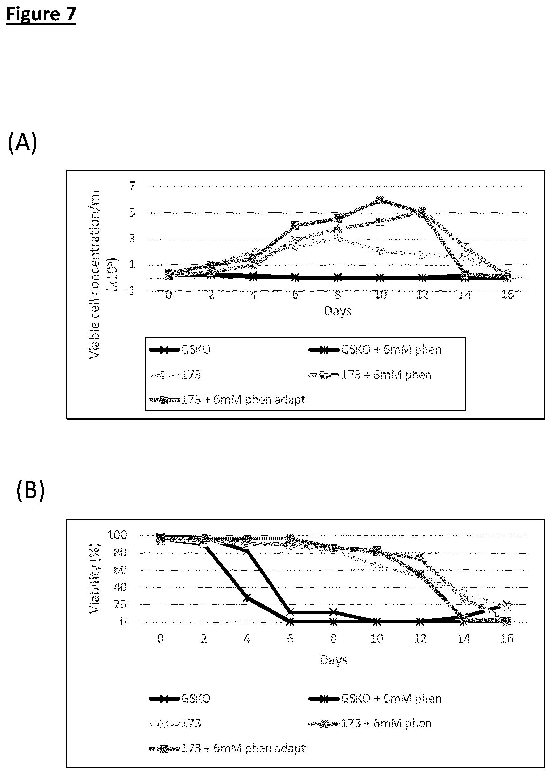

This example shows that growth is improved by preadapting exemplary production cell pools to additional supplemented phenylalanine prior to carrying out a batch culture (Fig. 7). Human truncated PAH expressing cells respond better to phenylalanine supplementation than the CHO version. Human truncated PAH expressing cells (LMM173) were preadapted by passaging cells with 6 mM phenylalanine prior to starting the growth curve. Cells were cultured in 125 ml Erlenmeyer flasks for 16 days. These cells were analysed for cell growth as measured by viable cell concentration and culture viability. Cell growth was enhanced by addition of phenylalanine but the growth lag phase was further reduced when cells were preadapted to growing in CD CHO no tyrosine but supplemented with 6 mM L-glutamine and 6 mM phenylalanine (Sigma P5482). GS-KO host cells were unable to grow in CD CHO medium supplemented with or without 6 mM phenylalanine.

### Example 9: Dual metabolic selection marker with recombinant protein production.

This example evaluates how the truncated PAH/GCH1 combined selection is exploitable when combined with a cell line producing a recombinant protein under glutamine synthetase selection. The promoter strength was varied driving GCH1 expression to determine if this impacted the subsequent cells that emerged in terms of the grow profile. Different plasmids which combinations of promoter and truncated PAH with GCH1 was the best combination for achieving maximum growth (highest viable cell concentration). The vectors used to generate these pools contained a truncated version of either CHO or human PAH (cassette 1, SV40 promoter), GCH1 (cassette 3 driven by either PGK, SV40 or mCMV promoter) and eGFP (cassette 2, driven by a CMV promoter) - see Table 1.

These were linearized and transfected into a cell line expressing the model monoclonal antibody cB72.3 under GS selection. Transfection was carried out in CD-CHO no glutamine and no tyrosine into T25 static flasks. Once cells had recovered and grown out after transfection and selection, these were transferred to shake flask in CM76 no glutamine and no tyrosine.

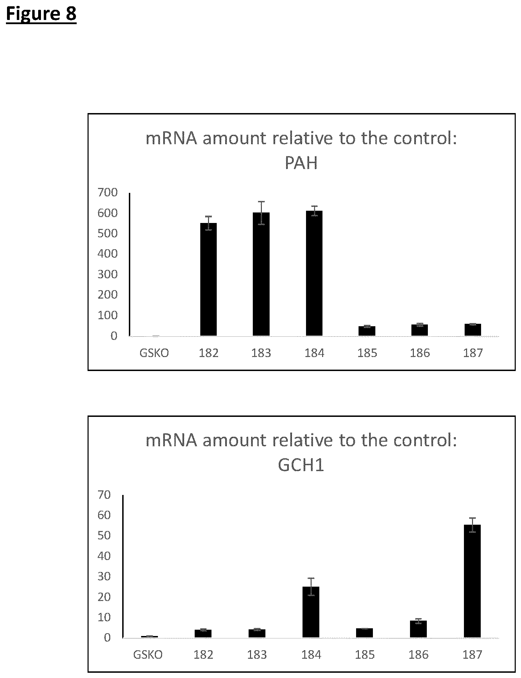

Fig. 8 shows qRT-PCR data of the expression of truncated CHO PAH and truncated human PAH mRNA expression in the resultant cell pools, and was comparable to findings in Example 4. GCH1 expression was also detected and the levels reflected the strength of the promoter which was driving the cassette. Analysis confirms mRNA overexpression of PAH in the truncated PAH cell pools. As previously observed the truncated CHO PAH was expressed at a much higher level than human PAH. GCH1 mRNA expression levels correlated with promoter strength driving the gene.

Western blot analysis of lysate from the cell pools described above was performed. All dual selection cell pools were grown in CM76 no glutamine and no tyrosine. The CHOK1SV GS-KO^{™} control sample was harvested from cells grown in complete medium (containing glutamine and tyrosine). Truncated PAH, GCH1 and GS were all detected in the dual selection marker expressing cell lines, except for CHO PAH since as per example 4 the antibody does not detect it (data not shown). A heavy chain antibody was also used to confirm that recombinant protein (cB72.3) was being secreted into the supernatant (data not shown). Tubulin, β-actin and L7a served as loading controls.

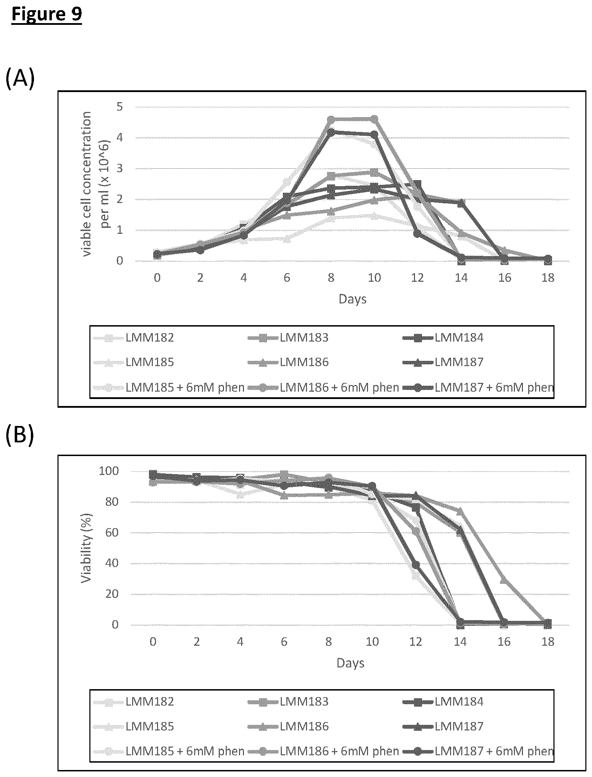

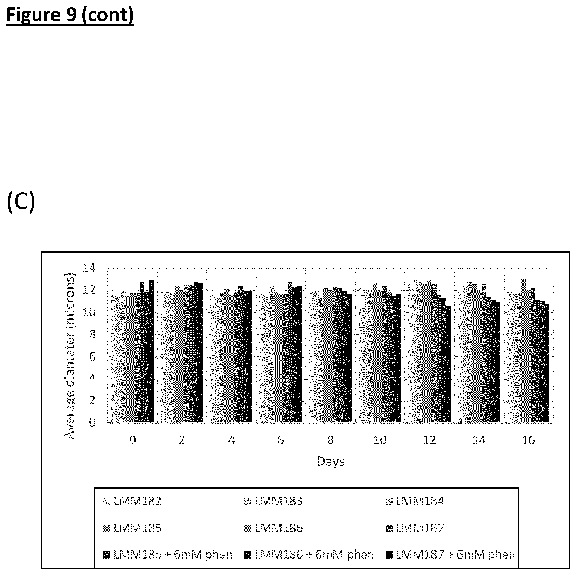

Fig. 9 shows growth data of exemplary production cell pools. Cell pools were cultured in 125 ml Erlenmeyer flasks for 18 days in the absence of tyrosine and glutamine and supplemented with additional phenylalanine as indicated. Every two days, the cells were sampled and the number of viable cells and culture viability assessed using a ViCell instrument; no further feeds were introduced. Maximum growth (achieving the highest viable cell concentration) was observed with LMM186 (SV40 human PAH, SV40 GCH1) when preadapted to an additional 6 mM phenylalanine supplemented medium.

Cells were cultured in CM76 without tyrosine or glutamine as two selection markers were being simultaneous utilised. The best growing cells pools were generated from LMM186 when supplemented with 6mM phenylalanine (SV40 PAH human & SV40 GCH1).

These results demonstrate that two different amino acid-based selection systems can be combined without any negative impact on cell line performance: the resulting cells demonstrate excellent growth characteristics. This will provide greater flexibility for expression since for example, one selection system can be used to make and maintain an engineered, stable cell line with a gene product that modifies cell line performance, whilst the other selection system can be used to introduce and maintain sequence(s) that encode a product that it is desired to manufacture.

The results also support the findings in Example 7 that using human PAH together with phenylalanine supplementation achieves superior performance.

## Claims

1. A method of selecting a eukaryotic cell comprising a nucleic acid sequence encoding a product of interest, the method comprising:
i) contacting a population of cells that are unable to survive or grow in the absence of tyrosine, with a vector system comprising
a) a first nucleic acid sequence comprising a sequence encoding a phenylalanine hydroxylase (PAH) which lacks a functional N-terminal regulatory domain such that the PAH enzyme molecule is constitutively active, operably linked to a first control sequence which enables expression of the PAH in a host cell, wherein the PAH is CHO PAH or human PAH;
b) a second nucleic acid sequence comprising a sequence encoding a GTP cyclohydrolase 1 (GCH1) operably linked to a second control sequence which enables expression of the GCH1 in a host cell; and
c) a third nucleic acid sequence comprising a sequence encoding a product of interest operably linked to a third control sequence which enables expression of the product in a host cell,
wherein
(1.) (a), (b) and (c) are present in the same vector; or
(2.) the vector system comprises two vectors wherein (c) is present in the same vector as (a) or (b),
under conditions that permit uptake of the vector system by the cells;
ii) culturing the cells in the presence of phenylalanine and under conditions where the level of tyrosine is lower than the level required for survival or growth of cells that do not express the PAH and GCH1 enzymes encoded by the vector system; and
iii) selecting one or more cells that are able to grow under such conditions to obtain one or more cells which contain the nucleic acid sequence encoding the product.

2. The method according to claim 1, wherein the eukaryotic cell is a mammalian cell, for example a CHO cell.

3. The method according to claim 1 or claim 2, wherein the cell culture medium lacks tyrosine.

4. A eukaryotic host cell comprising:
a) a first exogenous nucleic acid comprising a sequence which encodes a phenylalanine hydroxylase (PAH) which lacks a functional N-terminal regulatory domain such that the PAH enzyme molecule is constitutively active, operably linked to a first control sequence which enables expression of the PAH in the host cell, wherein the PAH is CHO PAH or human PAH;
b) a second exogenous nucleic acid which encodes a GTP cyclohydrolase 1 (GCH1), operably linked to a second control sequence which enables expression of the GCH1 in the host cell; and
c) a third exogenous nucleic acid which encodes a recombinant product of interest, operably linked to a third control sequence which enables expression of the recombinant product in the host cell, which third exogenous nucleic acid is present in the same exogenous nucleic acid sequence as the first and/or second exogenous nucleic acid.

5. The method according to claims 1 to 3, or the host cell according to claim 4, wherein the PAH has a deletion of the N-terminal regulatory domain.

6. The host cell according to any one of claims 4 to 5, which is a mammalian cell, for example a CHO cell.

7. The host cell according to any one of claims 4 to 6 wherein the first, second and third nucleic acid molecules are integrated into the genome of the host cell.

8. The host cell according to any one of claims 4 to 6 wherein the activity of the cell's endogenous genes encoding PAH and/or GCH1 has been reduced or abolished.

9. A vector system comprising one or more nucleic acid vectors comprising:
a) a first nucleic acid sequence comprising a sequence encoding a phenylalanine hydroxylase (PAH) which lacks a functional N-terminal regulatory domain such that the PAH enzyme molecule is constitutively active, operably linked to a first control sequence which enables expression of the PAH in a host cell, wherein the PAH is CHO PAH or human PAH;
b) a second nucleic acid sequence comprising a sequence encoding a GTP cyclohydrolase 1 (GCH1) operably linked to a second control sequence which enables expression of the GCH1 in a host cell; and
c) a multiple cloning site for inserting a sequence encoding a product of interest operably linked to a third control sequence which enables expression of the product in a host cell, wherein the multiple cloning site and third control sequence are present in the same vector as (a) and/or (b).

10. A vector system comprising one or more nucleic acid vectors comprising:
a) a first nucleic acid sequence comprising a sequence encoding a phenylalanine hydroxylase (PAH) which lacks a functional N-terminal regulatory domain such that the PAH enzyme molecule is constitutively active, operably linked to a first control sequence which enables expression of the PAH in a host cell, wherein the PAH is CHO PAH or human PAH;
b) a second nucleic acid sequence comprising a sequence encoding a GTP cyclohydrolase 1 (GCH1) operably linked to a second control sequence which enables expression of the GCH1 in a host cell; and
c) a third nucleic acid sequence comprising a sequence encoding an antibody operably linked to a third control sequence which enables expression of the product in a host cell, which third nucleic acid sequence is present in the same vector as (a) and/or (b).

11. A method of making a product, the method comprising culturing the host cell according to any one of claims 4 to 6 under conditions suitable for expressing the product, and recovering the product, and optionally subjecting the recovered product to one or more treatment or purification steps, wherein the cells are cultured in the presence of phenylalanine and under conditions where the level of tyrosine is lower than the level required for survival or growth of cells that do not express the PAH and GCH1 enzymes encoded by the vector system defined in any one of claims 1, 2 or 5.

## Patentansprüche

1. Verfahren zum Auswählen einer eukaryotischen Zelle, die eine Nukleinsäuresequenz umfasst, die ein Produkt von Interesse kodiert, wobei das Verfahren Folgendes umfasst:
i) Inkontaktbringen einer Population von Zellen, die in Abwesenheit von Tyrosin nicht in der Lage sind, zu überleben oder zu wachsen, mit einem Vektorsystem, umfassend
a) eine erste Nukleinsäuresequenz, die eine Sequenz umfasst, die eine Phenylalaninhydroxylase (PAH) kodiert, der eine funktionelle N-terminale regulatorische Domäne derart fehlt, dass das PAH-Enzymmolekül konstitutiv aktiv ist, funktionell verknüpft mit einer ersten Kontrollsequenz, die die Expression der PAH in einer Wirtszelle ermöglicht, wobei die PAH CHO-PAH oder humane PAH ist;
b) eine zweite Nukleinsäuresequenz, die eine Sequenz umfasst, die eine GTP-Cyclohydrolase 1 (GCH1) kodiert, funktionell verknüpft mit einer zweiten Kontrollsequenz, die die Expression der GCH1 in einer Wirtszelle ermöglicht; und
c) eine dritte Nukleinsäuresequenz, die eine Sequenz umfasst, die ein Produkt von Interesse kodiert, funktionell verknüpft mit einer dritten Kontrollsequenz, die die Expression des Produkts in einer Wirtszelle ermöglicht,
wobei
(1.) (a), (b) und (c) in dem gleichen Vektor vorhanden sind; oder
(2.) das Vektorsystem zwei Vektoren umfasst, wobei (c) in dem gleichen Vektor wie (a) oder (b) vorhanden ist,
unter Bedingungen, die die Aufnahme des Vektorsystems durch die Zellen erlauben;
ii) Kultivieren der Zellen in Gegenwart von Phenylalanin und unter Bedingungen, bei denen der Tyrosingehalt niedriger ist als der für das Überleben oder Wachstum von Zellen erforderliche Gehalt, die die von dem Vektorsystem kodierten PAH- und GCH1-Enzyme nicht exprimieren; und
iii) Auswählen einer oder mehrerer Zellen, die in der Lage sind, unter solchen Bedingungen zu wachsen, um eine oder mehrere Zellen zu erhalten, die die Nukleinsäuresequenz enthalten, die das Produkt kodiert.

2. Verfahren nach Anspruch 1, wobei die eukaryotische Zelle eine Säugetierzelle ist, beispielsweise eine CHO-Zelle.

3. Verfahren nach Anspruch 1 oder Anspruch 2, wobei dem Zellkulturmedium Tyrosin fehlt.

4. Eukaryotische Wirtszelle, umfassend:
a) eine erste exogene Nukleinsäure, die eine Sequenz umfasst, die eine Phenylalaninhydroxylase (PAH) kodiert, der eine funktionelle N-terminale regulatorische Domäne derart fehlt, dass das PAH-Enzymmolekül konstitutiv aktiv ist, funktionell verknüpft mit einer ersten Kontrollsequenz, die die Expression der PAH in der Wirtszelle ermöglicht, wobei die PAH CHO-PAH oder humane PAH ist;
b) eine zweite exogene Nukleinsäure, die eine GTP-Cyclohydrolase 1 (GCH1) kodiert, funktionell verknüpft mit einer zweiten Kontrollsequenz, die die Expression der GCH1 in der Wirtszelle ermöglicht; und
c) eine dritte exogene Nukleinsäure, die ein rekombinantes Produkt von Interesse kodiert, funktionell verknüpft mit einer dritten Kontrollsequenz, die die Expression des rekombinanten Produkts in der Wirtszelle ermöglicht, wobei die dritte exogene Nukleinsäure in der gleichen exogenen Nukleinsäuresequenz wie die erste und/oder zweite exogene Nukleinsäure vorhanden ist.

5. Verfahren nach Ansprüchen 1 bis 3 oder die Wirtszelle nach Anspruch 4, wobei die PAH eine Deletion der N-terminalen regulatorischen Domäne aufweist.

6. Wirtszelle nach einem der Ansprüche 4 bis 5, die eine Säugetierzelle ist, beispielsweise eine CHO-Zelle.

7. Wirtszelle nach einem der Ansprüche 4 bis 6, wobei das erste, zweite und dritte Nukleinsäuremolekül in das Genom der Wirtszelle integriert sind.

8. Wirtszelle nach einem der Ansprüche 4 bis 6, wobei die Aktivität der endogenen Gene der Zelle, die PAH und/oder GCH1 kodieren, reduziert oder beseitigt wurde.

9. Vektorsystem, umfassend einen oder mehrere Nukleinsäurevektoren, umfassend:
a) eine erste Nukleinsäuresequenz, die eine Sequenz umfasst, die eine Phenylalaninhydroxylase (PAH) kodiert, der eine funktionelle N-terminale regulatorische Domäne derart fehlt, dass das PAH-Enzymmolekül konstitutiv aktiv ist, funktionell verknüpft mit einer ersten Kontrollsequenz, die die Expression der PAH in einer Wirtszelle ermöglicht, wobei die PAH CHO-PAH oder humane PAH ist;
b) eine zweite Nukleinsäuresequenz, die eine Sequenz umfasst, die eine GTP-Cyclohydrolase 1 (GCH1) kodiert, funktionell verknüpft mit einer zweiten Kontrollsequenz, die die Expression der GCH1 in einer Wirtszelle ermöglicht; und
c) eine multiple Klonierungsstelle zum Einfügen einer Sequenz, die ein Produkt von Interesse kodiert, funktionell verknüpft mit einer dritten Kontrollsequenz, die die Expression des Produkts in einer Wirtszelle ermöglicht, wobei die multiple Klonierungsstelle und die dritte Kontrollsequenz in dem gleichen Vektor wie (a) und/oder (b) vorhanden sind.

10. Vektorsystem, umfassend einen oder mehrere Nukleinsäurevektoren, umfassend:
a) eine erste Nukleinsäuresequenz, die eine Sequenz umfasst, die eine Phenylalaninhydroxylase (PAH) kodiert, der eine funktionelle N-terminale regulatorische Domäne derart fehlt, dass das PAH-Enzymmolekül konstitutiv aktiv ist, funktionell verknüpft mit einer ersten Kontrollsequenz, die die Expression der PAH in einer Wirtszelle ermöglicht, wobei die PAH CHO-PAH oder humane PAH ist;
b) eine zweite Nukleinsäuresequenz, die eine Sequenz umfasst, die eine GTP-Cyclohydrolase 1 (GCH1) kodiert, funktionell verknüpft mit einer zweiten Kontrollsequenz, die die Expression der GCH1 in einer Wirtszelle ermöglicht; und
c) eine dritte Nukleinsäuresequenz, die eine Sequenz umfasst, die einen Antikörper kodiert, funktionell verknüpft mit einer dritten Kontrollsequenz, die die Expression des Produkts in einer Wirtszelle ermöglicht, wobei die dritte Nukleinsäuresequenz in dem gleichen Vektor wie (a) und/oder (b) vorhanden ist.

11. Verfahren zum Herstellen eines Produkts, wobei das Verfahren das Kultivieren der Wirtszelle nach einem der Ansprüche 4 bis 6 unter Bedingungen umfasst, die zum Exprimieren des Produkts geeignet sind, und das Gewinnen des Produkts und gegebenenfalls das Unterziehen des gewonnenen Produkts einem oder mehreren Behandlungs- oder Reinigungsschritten, wobei die Zellen in Gegenwart von Phenylalanin und unter Bedingungen kultiviert werden, bei denen der Tyrosingehalt niedriger ist als der für das Überleben oder Wachstum von Zellen erforderliche Gehalt, die die durch das in einem der Ansprüche 1, 2 oder 5 definierte Vektorsystem kodierten PAH- und GCH1-Enzyme nicht exprimieren.

## Revendications

1. Procédé de sélection d'une cellule eucaryote comprenant une séquence d'acide nucléique codant pour un produit d'intérêt, le procédé comprenant :
i) la mise en contact d'une population de cellules qui sont incapables de survivre ou de croître en l'absence de tyrosine, avec un système vectoriel comprenant
a) une première séquence d'acide nucléique comprenant une séquence codant pour une phénylalanine hydroxylase (PAH) qui ne possède pas de domaine régulateur N-terminal fonctionnel de telle sorte que la molécule d'enzyme PAH soit constitutivement active, liée de manière opérationnelle à une première séquence de contrôle qui permet l'expression de la PAH dans une cellule hôte, dans lequel la PAH est une CHO PAH ou une PAH humaine ;
b) une deuxième séquence d'acide nucléique comprenant une séquence codant pour une GTP cyclohydrolase 1 (GCH1) liée de manière fonctionnelle à une seconde séquence de contrôle qui permet l'expression de la GCH1 dans une cellule hôte ; et
c) une troisième séquence d'acide nucléique comprenant une séquence codant pour un produit d'intérêt liée de manière fonctionnelle à une troisième séquence de contrôle qui permet l'expression du produit dans une cellule hôte,
dans lequel
(1.) (a), (b) et (c) sont présents dans le même vecteur ; ou
(2.) le système vectoriel comprend deux vecteurs, dans lequel (c) est présent dans le même vecteur que (a) ou (b),
dans des conditions permettant l'absorption du système vectoriel par les cellules ;
ii) la culture des cellules en présence de phénylalanine et dans des conditions dans lesquelles le niveau de tyrosine est inférieur au niveau requis pour la survie ou la croissance de cellules qui n'expriment pas les enzymes PAH et GCH1 codées par le système vectoriel ; et
iii) la sélection d'une ou plusieurs cellules qui sont capables de croître dans ces conditions pour obtenir une ou plusieurs cellules qui contiennent la séquence d'acide nucléique codant pour le produit.

2. Procédé selon la revendication 1, dans lequel la cellule eucaryote est une cellule de mammifère, par exemple une cellule CHO.

3. Procédé selon la revendication 1 ou la revendication 2, dans lequel le milieu de culture cellulaire est dépourvu de tyrosine.

4. Cellule hôte eucaryote, comprenant :
a) un premier acide nucléique exogène comprenant une séquence qui code pour une phénylalanine hydroxylase (PAH) qui ne possède pas de domaine régulateur N-terminal fonctionnel de telle sorte que la molécule d'enzyme PAH soit constitutivement active, liée de manière opérationnelle à une première séquence de contrôle qui permet l'expression de la PAH dans la cellule hôte, dans lequel la PAH est une CHO PAH ou une PAH humaine ;
b) un deuxième acide nucléique exogène qui code pour une GTP cyclohydrolase 1 (GCH1) liée de manière fonctionnelle à une seconde séquence de contrôle qui permet l'expression de la GCH1 dans la cellule hôte ; et
c) un troisième acide nucléique exogène qui code pour un produit recombinant d'intérêt, lié de manière fonctionnelle à une troisième séquence de contrôle qui permet l'expression du produit recombinant dans la cellule hôte, lequel troisième acide nucléique exogène est présent dans la même séquence d'acide nucléique exogène que le premier et/ou deuxième acide nucléique exogène.

5. Procédé selon les revendications 1 à 3, ou cellule hôte selon la revendication 4, dans lequel la PAH présente une délétion du domaine régulateur N-terminal.

6. Cellule hôte selon l'une quelconque des revendications 4 à 5, qui est une cellule de mammifère, par exemple une cellule CHO.

7. Cellule hôte selon l'une quelconque des revendications 4 à 6, dans laquelle les première, deuxième et troisième molécules d'acide nucléique sont intégrées dans le génome de la cellule hôte.

8. Cellule hôte selon l'une quelconque des revendications 4 à 6, dans laquelle l'activité des gènes endogènes de la cellule codant pour la PAH et/ou la GCH1 a été réduite ou supprimée.

9. Système vectoriel comprenant un ou plusieurs vecteurs d'acide nucléique comprenant :
a) une première séquence d'acide nucléique comprenant une séquence codant pour une phénylalanine hydroxylase (PAH) qui ne possède pas de domaine régulateur N-terminal fonctionnel de telle sorte que la molécule d'enzyme PAH soit constitutivement active, liée de manière opérationnelle à une première séquence de contrôle qui permet l'expression de la PAH dans une cellule hôte, dans lequel la PAH est une CHO PAH ou une PAH humaine ;
b) une deuxième séquence d'acide nucléique comprenant une séquence codant pour une GTP cyclohydrolase 1 (GCH1) liée de manière fonctionnelle à une seconde séquence de contrôle qui permet l'expression de la GCH1 dans une cellule hôte ; et
c) un site de clonage multiple pour insérer une séquence codant pour un produit d'intérêt lié de manière fonctionnelle à une troisième séquence de contrôle qui permet l'expression du produit dans une cellule hôte, dans lequel le site de clonage multiple et la troisième séquence de contrôle sont présents dans le même vecteur que (a) et/ou (b).

10. Système vectoriel comprenant un ou plusieurs vecteurs d'acide nucléique comprenant :
a) une première séquence d'acide nucléique comprenant une séquence codant pour une phénylalanine hydroxylase (PAH) qui ne possède pas de domaine régulateur N-terminal fonctionnel de telle sorte que la molécule d'enzyme PAH soit constitutivement active, liée de manière opérationnelle à une première séquence de contrôle qui permet l'expression de la PAH dans une cellule hôte, dans lequel la PAH est une CHO PAH ou une PAH humaine ;
b) une deuxième séquence d'acide nucléique comprenant une séquence codant pour une GTP cyclohydrolase 1 (GCH1) liée de manière fonctionnelle à une seconde séquence de contrôle qui permet l'expression de la GCH1 dans une cellule hôte ; et
c) une troisième séquence d'acide nucléique comprenant une séquence codant pour un anticorps liée de manière fonctionnelle à une troisième séquence de contrôle qui permet l'expression du produit dans une cellule hôte, laquelle troisième séquence d'acide nucléique est présente dans le même vecteur que (a) et/ou (b).

11. Procédé de fabrication d'un produit, le procédé comprenant une mise en culture de la cellule hôte selon l'une quelconque des revendications 4 à 6 dans des conditions appropriées à l'expression du produit, et une récupération du produit, et facultativement une soumission du produit récupéré à une ou plusieurs étapes de traitement ou de purification, dans lequel les cellules sont cultivées en présence de phénylalanine et dans des conditions dans lesquelles le niveau de tyrosine est inférieur au niveau requis pour la survie ou la croissance de cellules qui n'expriment pas les enzymes PAH et GCH1 codées par le système vectoriel défini dans l'une quelconque des revendications 1, 2 ou 5.
